# FASCICULE DE BREVET EUROPEEN

(11) **EP 1 196 371 B1**
(45) Date de publication et mention de la délivrance du brevet: **02.11.2005**
(21) Numéro de dépôt: 00958598.5
(22) Date de dépôt: 21.07.2000
(51) Int. Cl.: C07C 211/09, C07C 327/30, C07C 323/27, C07C 323/59, C07D 277/22, C07D 277/24, C07D 277/30, C07D 295/14, A61K 31/14, A61K 31/145, A61K 31/425, A61P 33/06, C07D 327/06

(54) **PRECURSEURS DE SELS DE BIS-AMMONIUM QUATERNAIRE ET LEURS APPLICATIONS COMME PRODROGUES AYANT UNE ACTIVITE ANTI-PARASITAIRE**
VORPRODUKTE VON BIS-QUATERNÄREN AMMONIUMSALZEN UND DEREN VERWENDUNG ALS PRODRUGS MIT ANTI-PARASITÄRER WIRKUNG
QUATERNARY BIS-AMMONIUM SALT PRECURSORS AND THEIR USES AS PRODRUGS HAVING AN ANTIPARASITIC ACTIVITY

(30) Priorité: 21.07.1999 FR 9909471
(43) Date de publication de la demande: 17.04.2002
(73) Titulaire: CENTRE NATIONAL DE LA RECHERCHE SCIENTIFIQUE (CNRS), 75794 Paris Cedex 16 (FR)
(72) Inventeur: VIAL, Henri, F-34060 Montpellier (FR); CALAS, Michèle, F-34090 Montpellier (FR); ANCELIN, Marie-Laure, F-34270 St Jean de Cuculles (FR); BOURGUIGNON, Jean-Jacques, F-67150 Hipsheim (FR); VIDAL, Valérie, 34090 Montpellier (FR); RUBI, Eric, 95370 Montigny les Cormeilles (FR)
(74) Mandataire: Peaucelle, Chantal
(86) Numéro de dépôt international: PCT/FR2000/002122
(87) Numéro de publication internationale: WO 2001/005742

(56) Documents cités:
- FR-A- 2 751 967
- US-A- 3 131 220
- US-A- 3 278 537
- MARTI, JOSEP ET AL: "Introduction to a rational design of chiral thiazolium salts" TETRAHEDRON LETT. (1993), 34(3), 521-4, XP002141680
- CHEMICAL ABSTRACTS, vol. 55, no. 12, 12 juin 1961 (1961-06-12) Columbus, Ohio, US; R.R. MITCHELL: "The intestinal absorption of some omega-haloalkylamines and their quaternary analogs" colonne 11643g; XP002141681 & J. PHARMACOL. EXPTL. THERAP., vol. 131, 1961, pages 334-340,
- F. LOPEZ-CALAHORRA ET AL.: "Use of 3,3'-polymethylene-bridged thazolium salts plus bases as catalysts of benzoin condensation and its mechanistic implications: Proposal of a new mechanism in aprotic conditions" HETEROCYCLES, vol. 37, no. 3, 1994, pages 1570-1597, XP002141688
- D.D.LIBMAN ET AL.: "Somes bisquaternary salts" JOURNAL OF THE CHEMICAL SOCIETY, 1952, pages 2305-2307, XP002141689 LETCHWORTH GB

## Description

L'invention a pour objet des précurseurs de sels de bis-amonium quaternaire et leurs applications en tant que prodrogues présentant, en particulier, un effet anti-parasitaire, et plus spécialement antipaludique.

L'extension géographique des maladies parasitaires, et tout spécialement du paludisme, est considérable.

Plus de 100 pays sont touchés à ce jour par le paludisme et plus de 2 milliards de personnes sont exposées au risque d'infection, soit près de la moitié de la population mondiale (pour la situation du paludisme dans le monde, voit Butler et al., Nature, 1997, 386, 535-540).

La recrudescence de souches chimiorésistantes de *plasmodium falciparum* (l'espèce mortelle pour l'homme) en Asie, en Afrique et en Amérique latine est plus que jamais d'actualité et limite considérablement l'efficacité des traitements disponibles.

On mesure ainsi l'urgence de disposer de médicaments anti-paludiques efficaces.

Dans Tetrahedron Letters, 1993, p.521-524, les auteurs décrivent l'utilisation de sels de thiazolium chiraux en tant que catalyseurs, pour induire une asymétrie dans la condensation de la benzoïne.

US-A-3278537 décrit des dérivés de thiamine et leur préparation.

US-A-3131220 se rapporte à des sels de diméthylène-diamine tétrasubstitués et enseigne leur effet hypotenseur.

L'abrégé CA. Vol.55-n°12, 1961 décrit l'absorption intestinale d'oméga-haloalkylamines et de leurs analogues quaternaires.

Dans Hétérocycles, vol. 37, n°3, 1994, les auteurs rapportent l'utilisation de sels de thiazolium et de benzothiazolium reliés en 3,3' par un pont polyméthylène.

Dans Journal of Chenical Society, 1952 p.2305-2307, les auteurs décrivent des sels biquaternaires de thiazoline.

Dans des travaux précédents, certains des co-inventeurs de la présente demande de brevet ont développé un modèle pharmacologique original capable d'empêcher la reproduction du parasite. Les composés synthétisés présentent une structure de type bis-ammonium quaternaire avec un bras espaceur, l'un des composés les plus étudiés étant constitué par le 1,16-hexadécaméthylène bis-(N-méthylpyrrolidinium), répondant à la formule

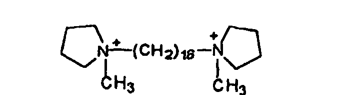

Ce composé sera appelé ci-après G25 (brevet FR 2 751 967).

Si de tels composés présentent un intérêt considérable compte tenu des guérisons qu'ils entraînent *in vivo*, sans rechutes, il s'avère toutefois que leur activité par voie orale est inférieure par un facteur d'au moins 100 à celle observée par voie intramusculaire.

La poursuite des travaux des inventeurs pour rechercher de nouveaux composés présentant une efficacité accrue lorsqu'on les administre par voie orale les a conduits à étudier une stratégie basée sur l'élaboration de prodrogues neutres, *a priori* plus facilement absorbables, capables de générer *in vivo* la drogue active qui se présente sous forme ionisée.

De manière surprenante, ces travaux ont permis de développer des prodrogues de sels de bis-ammonium quaternaire de grande efficacité, dotées d'une activité anti-parasitaire élevée, aisément absorbables, générant *in vivo* des drogues actives dont la biodisponibilité est élevée.

L'invention vise donc à fournir de nouveaux dérivés neutres, à activité antipaludique élevée, administrables aussi par voie orale, ainsi que des métabolites ionisés générés *in vivo*.

Elle vise également un procédé de synthèse de ces prodrogues.

Selon encore un autre aspect, l'invention vise la mise à profit des propriétés de ces prodrogues pour l'élaboration de principes actifs de médicaments utilisables pour le traitement des maladies parasitaires, et en particulier du paludisme et des babésioses animales ou humaines.

Les précurseurs de drogues à effet anti-paludique selon l'invention sont caractérisés en ce qu'il s'agit de produits capables de générer des sels de bis-ammonium quaternaire et qu'ils répondent à la formule générale (I) 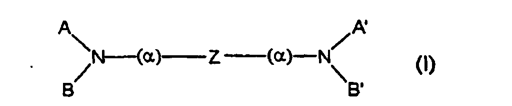 dans laquelle
- A et A' sont identiques ou différents l'un de l'autre et représentent
   . soit, respectivement, un groupe A₁ et A'₁ de formule 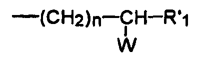 où n est un entier de 2 à 4 ; R'₁ représente un atome d'hydrogène, un radical alkyle en C1 à C5, éventuellement substitué par un radical aryle (notamment un radical phényle), un hydroxy, un alkoxy, dans lequel le radical alkyle comprend de 1 à 5 C, ou aryloxy (notamment phénoxy); et W représente un atome d'halogène choisi parmi le chlore, le brome ou l'iode, ou un groupe nucléofuge, comme le radical tosyle CH₃-C₆H₄-SO₃, mésityle CH₃-SO₃, CF₃-SO₃, NO₂-C₆H₄-SO₃,
   . soit un groupe A₂ qui représente un radical formyle -CHO, ou acétyl -COCH₃,
- B et B' sont identiques ou différents l'un de l'autre et représentent
   . soit respectivement les groupes B₁ et B'₁, si A et A' représentent respectivement A₁ et A'₁ , B₁ et B'₁ représentant un groupe R₁ qui présente la même définition que R'₁ ci-dessus, mais ne peut pas être un atome d'hydrogène,
   . soit respectivement les groupes B₂ et B'₂, si A et A' représentent A₂, B₂ ou B'₂ étant le groupe R₁ tel que défini ci-dessus, ou un groupement de formule 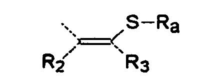 dans lequel -Ra représente un groupe RS- ou RCO-, où R est un radical alkyle en C1 à C6, notamment de C1 à C5, linéaire, ramifié ou cyclique, le cas échéant substitué par un ou plusieurs groupements hydroxy ou alcoxy, ou aryloxy ou un groupe amino et/ou un groupe - COOH ou COOM, où M est un alkyle en C1 à C3; un radical phényle ou benzyle, dans lequel le radical phényle est le cas échéant substitué par au moins un radical alkyle ou alcoxy en C1 à C5, ceux-ci étant éventuellement substitués par un groupe amino, ou par un hétérocycle azoté ou oxygéné, un groupe -COOH ou -COOM; ou un groupe -CH₂-hétérocycle, à 5 ou 6 éléments, azoté et/ou oxygéné ; R₂ représente un atome d'hydrogène, un radical alkyle en C1 à C5, ou un groupe -CH₂-COO-alkyl(C1 à C5); et R₃ représente un atome d'hydrogène, un radical alkyle ou alkényle en C1 à C5, le cas échéant substitué par -OH, un groupement phosphate, un radical alkoxy, dans lequel le radical alkyle est en C1 à C3, ou aryloxy, ou un groupe alkyl (ou aryl) carbonyloxy; ou encore R₂ et R₃ forment ensemble un cycle à 5 ou 6 atomes de carbone ; R et R₃ peuvent être reliés pour former un cycle de 5 à 7 atomes (carbone, oxygène, soufre)
- α représente
   . soit une simple liaison, lorsque A et A' représentent A₁ et A'₁ : ou lorsque A et A' représentent A₂ , c'est-à-dire un groupe -CHO ou -COCH₃, et B₂ et B'₂ représentent 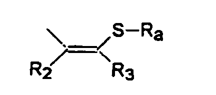
   . soit, lorsque A et A' représentent A₂ et B₂ et B'₂ représentent R₁ , un groupement de formule 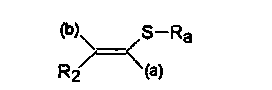 ou un groupement de formule 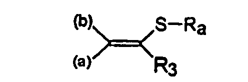 dans lesquels (a) représente une liaison vers Z et (b) une liaison vers l'atome d'azote,
- Z représente un radical alkyle en C6 à C21, notamment en C13 à C21, le cas échéant avec insertion d'une ou de plusieurs liaisons multiples, et/ou d'un ou plusieurs hétéroatomes O et/ou S, et/ou d'un ou de plusieurs cycles aromatiques, et les sels pharmaceutiquement acceptables de ces composés.

A moins de précisions contraires, "aryle" et "aromatique" tels qu'utilisés pour définir les produits de l'invention désignent un phényle ou tout cycle, ou hétérocycle, ayant un caractère aromatique comme les cycles pyridine, oxazole, thiazole, "alkényl" désigne un alkyl comportant une ou plusieurs insaturations, "groupe amino" désigne -NH₂ ou dialkyl (C₁-C₃) amino, et liaison multiple désigne une insaturation (double ou triple liaison) entre 2 atomes de carbone.

Un groupe préféré de composés selon l'invention est constitué par des haloalkylamines, précurseurs de sels de bis-ammonium quaternaire, qui répondent à la formule générale (II)

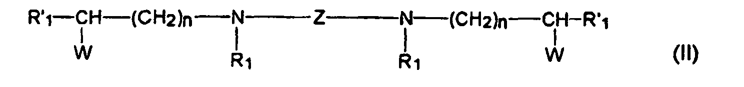

Dans ces composés, R₁, R'₁, W, n et Z sont tels que définis ci-dessus.

Dans une famille préférée de ces composés, Z représente un radical alkyle en C13 à C21.

Dans des dérivés préférés, Z représente un groupe -(CH₂)₁₆-_{.}

Dans un sous-groupe de cette famille, R₁ est avantageusement un radical méthyle.

Dans un autre sous-groupe, R₁ est un radical méthyle et R'₁ est soit un atome d'hydrogène, soit un radical méthyle, et W est un atome de chlore.

Des composés particulièrement préférés sont choisis parmi le dichlorhydrate du N,N'-diméthyl-N,N'-(5-chloropentyl)-1,16-hexadécanediamine, ou le dichlorhydrate du N,N'-diméthyl-N,N'-(4-chloropentyl)-1,16-hexadécanediamine.

Un autre groupe préféré de composés selon l'invention est constitué par des précurseurs de thiazolium et répondent à la formule générale (III) 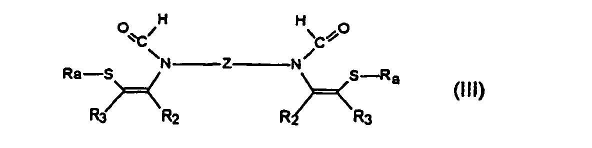 dans laquelle Rₐ, R₂, R₃, et Z sont tels que définis ci-dessus.

Dans un sous-groupe préféré de cette famille, Rₐ représente un radical RCO-. Des composés particulièrement préférés sont choisis parmi
le N,N'-diformyl-N,N'-di[1-méthyl-2-S-thiobenzoyl-4-méthoxybut-1-ényl]-1,12-diaminodoclécane (désigné dans les exemples par TE4c),
le N,N'-diformyl-N,N'-di[1-méthyl-2-S-(p-diéthylaminométhylphényl-carboxy)thio-4-méthoxybut-1-ényl]-1,12-diaminododécane (TE4f),
le N,N'-diformyl-N,N'-di[1-méthyl-2-S-(p-morpholino-méthylphénylcarboxy)-thio-4-méthoxybut-1-ényl]-1,12-diaminododécane (TE4g),
le N,N'-diformyl-N,N'-di[1-méthyl-2-S-thiobenzoyl-4-méthoxybut-1-ényl]-1,16-diaminohexadécane (TE8),
le N,N'-diformyl-N,N'-di[1(2-oxo-4,5-dihydro-2,3-oxathian-4-ylidène)éthyl]1,12-diaminododécane (TE3)

Dans un autre sous-groupe préféré, Rₐ représente un radical RS-. Des composés particulièrement préférés sont choisis parmi
le N,N'-diformyl-N,N'-di[1-méthyl-2-tétrahydrofurfuryl-méthyldithio-4-hydroxybut-1-ényl]-1,12-diaminododécane (TS3a),
le N,N'-diformyl-N,N'-di[1-méthyl-2-propyl-dithio-4-hydroxybut-1-ényl]-1,12-diaminododécane (TS3b),
le N,N'-diformyl-N,N'-di[1-méthyl-2-benzyl-dithio-4-hydroxybut-1-ényl]-1,12-diaminododécane (TS3c),
le N,N'-diformyl-N,N'-di[1-méthyl-2-(2-hydroxyéthyl)-dithio-4-hydroxybut-1-ényl]-1,12-diaminododécane (TS3d)
le N,N'-diformyl-N,N'-di[1-méthyl-2-propyldithio-4-méthoxybut-1-ényl]-1,12-diaminododécane (TS4b),
le N,N'-diformyl-N,N'-di[1-méthyl-2-propyldithio-éthényl]-1,12-diaminododécane (TS6b).

Un autre groupe préféré de composés selon l'invention est constitué aussi par des précurseurs de sels de thiazolium qui répondent à la formule générale (IV) 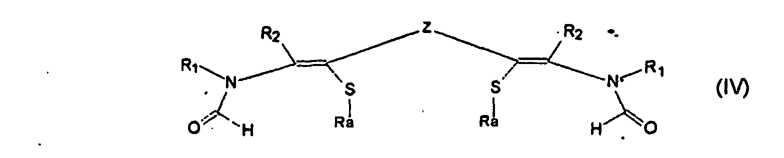 dans laquelle Rₐ, R₂, R₁ et Z sont tels que définis ci-dessus.

Des composés particulièrement préférés sont choisis parmi le 2,17-(N,N'-diformyl-N,N'-diméthyl)diamino-3,16-S-thio-p-méthoxybenzoyl-6,13-dioxaoctadéca-2',16-diène (TE9),
le 2,17 (N,N'-diformyl-N,N'-dibenzyl)diamino-3,16-S-thio-p-méthoxybenzoyl-6,13-dioxaoctadéca-2,16-diène (TE10),
le 3,18 (N,N'-diformyl-N,N'-diméthyldiamino-4,17-S-thiobenzoyl-eicosa-3,17-diénedioate d'éthyle (TE12),
le 3,18-(N,N'-diformyl-N,N'-dibenzyl)diamino-4,17-S-thiobenzoyl-eicosa-3,17-diènedioate d'éthyle (TE13).

Un autre groupe préféré de composés selon l'invention est constitué encore par des précurseurs de sels de thiazolium qui répondent à la formule générale (V) 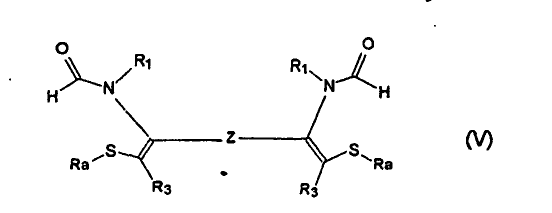 dans laquelle Rₐ, R₃, R₁ et Z sont tels que définis ci-dessus.

Des composés particulièrement préférés sont choisis parmi le 2,15-(N,N'-diformyl-N,N'-diméthyl)diamino-1,16-S-thio-benzoyl-hexadéca-1,15-diène (TE15),
le 2,15-(N,N'-diformyl-N,N'-dibenzyl)diamino-1,16-S-thio-benzoyl-hexadéca-1,15-diène (TE16).

Les précurseurs selon l'invention se présentent le cas échéant sous forme de sels. On citera à titre d'exemples les chlorhydrates, les citrates, les tartrates, les maléates ou les lactates.

L'invention vise également les dérivés cyclisés générés à partir des précurseurs de thiazolium définis ci-dessus.

Ces dérivés répondent à la formule générale (VI) 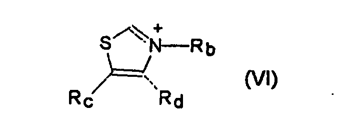 dans laquelle
. R_{b} représente R₁ ou T, T représentant le groupe de formule 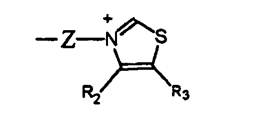
. R_{d} représente R₂ ou P, P représentant le groupe de formule 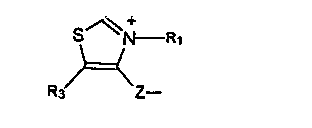
R_{c} représente R₃ ou U, U représentant le groupe de formule 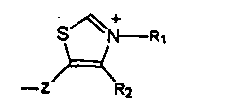 R₁, R₂, R₃ et Z étant tels que définis ci-dessus,
étant entendu que R_{b} = T si R_{c} = R₃ et R_{d} = R₂ ; R_{d} = P si R_{c} = R₃ et R_{b} = R₁ ; et R_{c} = U si R_{b} = R₁, et R_{d} = R₂.

Conformément à l'invention, les précurseurs de thiazolium de formule'générale (III) à (IV) définis ci-dessus peuvent être obtenus par un procédé caractérisé en ce qu'il comprend la réaction en milieu basique d'un dérivé de thiazole de formule (VI), comme illustré dans les exemples.

De manière avantageuse, pour obtenir Rₐ = RCO-, on fait réagir un dérivé de thiazolium de formule (VI) avec un dérivé RCOR', où R est tel que défini ci-dessus et R' est un atome d'halogène, et pour obtenir Rₐ = RS-on fait réagir lesdits dérivés de thiazolium de formule (VI) avec un dérivé de thiosulfate RS₂O₃Na.

La série N-dupliquée des sels de thiazolium est obtenue, de manière générale, en faisant réagir un dérivé de thiazole convenablement substitué avec un dihalogénure d'alkyle à reflux dans un solvant organique.

La série C dupliquée sur le carbone C5 du cycle thiazole, qui comporte un oxygène dans la chaîne Z, est obtenue par réaction d'un dérivé de thiazole de formule générale (VII) 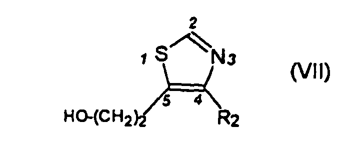 avec un dihalogénure d'alcane, en milieu basique, puis l'addition de R₁X, le milieu réactionnel étant avantageusement porté à reflux dans un solvant organique, notamment alcoolique comme l'éthanol, pendant une durée suffisante pour obtenir la quaternisation de l'atome d'azote du thiazole par fixation de R₁.

L'ouverture du cycle thiazolium obtenu est ensuite effectuée en milieu basique, et par action soit de R-COCl, soit de R-S₂O₃Na.

Pour obtenir la série C-dupliquée sur le carbone 5 du cycle thiazole et ne comportant pas d'oxygène dans la chaîne Z, on synthétise tout d'abord un composé de structure 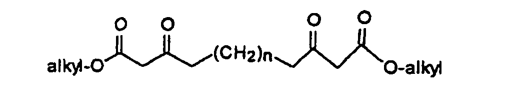 par réaction d'un acétoacétate d'alkyle avec NaH, dans un solvant organique, à une température de l'ordre de 0°C, puis on alkyle le composé formé avec par exemple un alkyllithium et on ajoute au milieu réactionnel un dihalogénoalcane.

Le composé obtenu est dibromé par addition de brome à une température de l'ordre de 0°C, puis additionné de thioformamide et laissé sous reflux plusieurs jours. Par addition de R₁X au mélange réactionnel, soumis ensuite à reflux pendant plusieurs jours, on obtient un thiazolium dont l'ouverture est ensuite réalisée en milieu basique.

Pour obtenir la série C-dupliquée sur le carbone C4 du cycle thiazole, et ne comportant pas d'oxygène dans la chaîne Z, on fait réagir un composé Z(CO-CH₂X)₂ avec CH(=S)NH₂, puis on ajoute R₁X.

L'invention vise également "un procédé d'obtention des haloalkylamines répondant à la formule générale (II). Ce procédé est caractérisé par l'alkylation d'un aminoalcool de formule 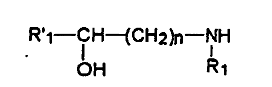 par un α, ω-dihalogénure d'alkyle X-Z-X, ce qui conduit à un bis-aminoalcool traité par un composé capable de libérer le groupe W.

L'alkylation de l'aminoalcool est réalisée par exemple avec un α, ω-dichlorure d'alkyle, de formule Cl-Z-C1, dans l'éthanol, en présence de diisopropyléthylamine, l'aminoalcool étant en large excès par rapport à l'halogénure (d'environ 2,1/1). Le bis-aminoalcool obtenu est ensuite traité par un composé capable de libérer W, qui peut être par exemple du chlorure de thionyle, dans du dichlorométhane, pour obtenir un composé dans lequel W représente C1, ou par le chlorure d'un acide sulfonique, par exemple le chlorure de tosyle pour obtenir un composé dans lequel W = CH₃-C₆H₄-SO₃-.

L'étude de l'activité des produits de l'invention vis-à-vis de parasites, et notamment de *Plasmodium,* a montré qu'ils présentent une forte activité *in vitro.*

Ainsi, les valeurs de CI₅₀ (concentration inhibant 50% du parasite) sont de l'ordre du nM vis-à-vis de *P. falciparum*.

L'invention vise donc la mise à profit des propriétés des précurseurs de l'invention et des composés cyclisés thiazolium pour l'élaboration de compositions pharmaceutiques.

Les compositions pharmaceutiques de l'invention sont caractérisées en ce qu'elles renferment une quantité efficace d'au moins un précurseur tel que défini ci-dessus, ou d'au moins un composé cyclisé de thiazolium, en association avec un véhicule pharmaceutique inerte ou d'au moins un composé cyclisé thiazolium.

L'invention vise également l'utilisation d'au moins l'un desdits précurseurs, ou d'au moins l'un desdits composés cyclisés de thiazolium, pour fabriquer des médicaments pour le traitement des maladies infectieuses, en particulier du paludisme ou des babésioses chez l'homme ou l'animal.

Ces compositions renferment le cas échéant des principes actifs d'autres médicaments. On citera notamment leur association avec d'autres antipaludiques (tels que les agents lysosomotropes, l'atovaquone, les antifoliques ou antifoliniques, ou l'artémisinine ou l'un de ses dérivés) pour raison de synergie pharmacologique ou d'évitement de résistance.

On les utilisera également avec avantage en association avec des composés facilitant leur assimilation.

Les compositions pharmaceutiques de l'invention sont administrables sous différentes formes, plus spécialement par voie orale ou injectable ou encore par voie rectale.

Pour l'administration par voie orale, on a recours en particulier à des comprimés, pilules, tablettes, gélules, gouttes.

D'autres formes d'administration comprennent des solutions injectables par voie intra-veineuse, sous-cutanée ou intra-musculaire, élaborées à partir de solutions stériles ou stérilisables. Il peut s'agir également de suspensions ou d'émulsions.

On peut également utiliser des suppositoires pour d'autres formes d'administration.

Les compositions de l'invention sont particulièrement appropriées pour le traitement des maladies infectieuses chez l'homme et l'animal, en particulier du paludisme ou des babésioses.

A titre indicatif, la posologie utilisable chez l'homme correspond aux doses suivantes : ainsi on administre par exemple au patient de 0,02 à 80 mg/kg en une ou plusieurs prises.

L'invention vise encore les réactifs biologiques renfermant comme principes actifs, les précurseurs de thiazolium définis plus haut.

Ces réactifs peuvent être utilisés comme références ou étalons dans des études d'éventuelles activités anti-parasitaires.

D'autres caractéristiques et avantages de l'invention apparaîtront dans les exemples qui suivent relatifs à l'obtention de précurseurs de thiazolium et à l'étude de leur activité anti-parasitaire. Dans ces exemples, il sera fait référence aux figures 1 à 8, qui représentent respectivement,
- la figure 1, l'activité antipaludique d'un précurseur de thiazolium (désigné par TE4c dans les exemples), et du thiazolium correspondant (T4) en fonction de la concentration en drogue, selon le test de Desjardins, (Desjardins R.E. et al, Antimicrob. Agents Chemother. 1979, 16, 710-718),
- les figures 2 et 3, la pharmacocinétique chez la souris d'un précurseur de thiazolium selon l'invention à faible dose (désigné par TE4c dans les exemples) après administration par voie ip et *per os,*
- la figure 4, une représentation semi-logarithmique de la pharmacocinétique chez la souris de précurseurs et de drogue selon l'invention, après administration par voie ip et *per os*,
- les figures 5A et 5B, la pharmacocinétique d'un précurseur et d'une drogue de l'invention chez la souris après administration par voie ip et *per os*,
- les figures 6 et 7, les pharmacocinétiques chez la souris après administration par voie ip *per os*, et
- les figures 8A et 8B, la pharmacocinétique de précurseurs de l'invention chez le singe.

Dans les différents schémas donnés dans les exemples, les substituants présentent les significations données ci-dessus, X représente un contre-ion,
Me = méthyle, Et = éthyle, Ph ou Φ = C₆H₅-,
DMSO=diméthylsulfoxyde, THF= tétrahydrofuranne, Bu = butyl.

### I. SYNTHESE DES PRECURSEURS DE THIAZOLIUM

### A. Synthèse des prodrogues disulfures (TS):

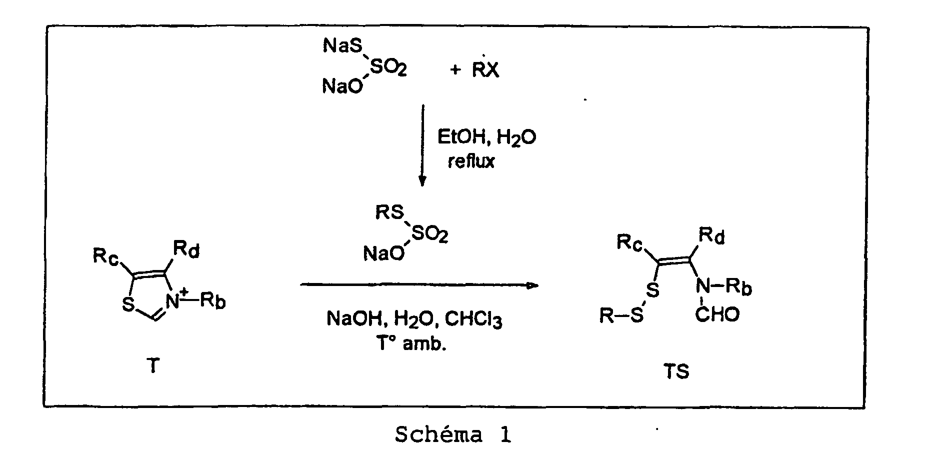

### Mode opératoire général:

### a) Synthèse du thiosulfate d'alkyle (sel de Bunte)

30 mmoles de dérivé halogéné sont dissous dans 15 mL d'éthanol et 5g (30 mmol) de thiosulfate de sodium dans le minimum d'eau sont ajoutés. Le mélange est chauffé à reflux pendant 5 jours. La solution est évaporée à sec et le residu brut obtenu est utilisé sans purification.

### b) Synthèse de la prodrogue disulfure

2,6 mmoles de drogue thiazolium sont dissous dans 10mL d'eau. 0,6g de NaOH puis 10mL de CHCl₃ sont ajoutés et le mélange est agité vigoureusement pendant 10mn. On ajoute ensuite goutte à goutte 7,8 mmoles de thiosulfate d'alkyle obtenu précédemment et le mélange est agité à température ambiante pendant 2 heures. La phase organique est séparée et lavée par une solution 5% HCl. Elle est ensuite neutralisée par une solution de NaHCO₃, séchée par du Na₂SO₄ et concentrée. L'huile obtenue est purifiée sur gel de silice en éluant par un mélange CH₂Cl₂/MeOH (9,5/0,5).

### 1. Synthèse du N,N'-diformyl-N,N'-di[1-méthyl-2-tétrahydro-furfurylméthyldithio-4-hydroxybut-1-ényl]-1,12-diaminododécane (TS3a)

### a) Préparation du thiosulfate de tétrahydrofurfurylméthyl

Il est réalisé selon le §a du mode opératoire général ci-dessus, à partir du chlorure de tétrahydrofurfuryle.
RMN ¹H (250 MHz, D₂O) : δ 4,04 (m, 1H, CH₂-C*H*-O), 3, 61 (m, 2H, -C*H*₂-O), 3,00(m,2H,S-C*H*₂-CH), 1,47-1,88(m,4H,-CH-C*H*₂-C*H*₂-)

### b) Synthèse de TS3a

Selon le §b du mode opératoire général ci-dessus, à partir de T3 (voir sa préparation plus loin) et du sel de Bunte obtenu précédemment, on obtient une huile jaunâtre.
RMN ¹H (250 MHz, CDCl₃) : δ 7,88-7,99(2s,2H,C*H*O), 3, 95 (m, 2H, O-C*H*-CH₂), 3, 7 (m, 8H, -C*H*₂-OH+CH-OC*H*₂-CH₂), 3,32(m,4H,S-C*H*₂-), 2,8(m,8H,N-C*H*₂-+=C-C*H*₂), 1, 64-1, 94 (m, 18H, -N-CH₂-C*H*₂-+-CH-C*H*₂-C*H*₂-+C*H*₃-C=), 1, 26 (m, 16H, (C*H*₂)₈).

### 2. Synthèse du N,N'-diformyl-N,N'-di[1-méthyl-2-propyl-dithio-4-hydroxybut-1-ényl]-1,12-diaminododécane (TS3b)

### a) Préparation du thiosulfate de propyle

Il est réalisé selon le §a du mode opératoire général ci-dessus, à partir du bromure de propyle.
RMN ¹H (250 MHz, D₂O) : δ 0,74 (t, 2H, -C*H*₃), 1,51 (m, 2H, -CH₂C*H*₂CH₃), 2,86 (t, 2H, S-C*H*₂-CH₂-)

### b) Préparation de TS3b

Selon le §b du mode opératoire général ci-dessus, à partir de T3 (voir sa préparation plus loin) et du sel de Bunte obtenu précédemment, on obtient une huile jaunâtre.
RMN ¹H (250 MHz, CDCl₃) : δ 7,91-7,99(2s,2H,C*H*O), 3,8(m,4H,-C*H*₂-OH), 3,39(m,4H,S-C*H*₂-), 2,91(t,4H, =C-C*H*₂), 2, 62 (t, 4H,N-C*H*₂-), 2, 00 (d, 6H, C*H*₃-C=), 1, 64 (m, 8H, -N-CH₂-C*H*₂-+-S-CH₂-C*H*₂-), 1,26(m,16H,-(C*H*₂)₈-), 0,97(t,6H,-S-CH₂-CH₂-C*H*₃).

### 3. Synthèse de N,N'-diformyl-N,N'-di[1-méthyl-2-benzyl-dithio-4-hydroxybut-1-ényl]-1,12-diaminododécane (TS3c)

### a) Préparation du thiosulfate de benzyle

Il est réalisé selon le § a du mode opératoire général ci-dessus, à partir du bromure de benzyle.
RMN ¹H (250 MHz, D₂O) : δ 4,13(s,2H,-C*H*_{*2*}-), 7,23(m,5H,-Ar*H*).

### b) Préparation de TS3c

Selon le § b du mode opératoire général ci-dessus, à partir de T3 (voir sa préparation plus loin) et du sel de Bunte obtenu précédemment, on obtient une huile jaunâtre.
RMN ¹H (250 MHz, CDCl₃) : δ 7,91-7,99(2s,2H,C*H*O), 3,89 (s,4H,S-C*H*₂-Ph), 3,73(t,4H,CH₂-OH) , 3,40(t,4H,N-C*H*₂-), 2,75(t,4H,=C-C*H*₂), 1,96(s,6H,C*H*₃-C=), 1,52(m,4H,-N-CH₂-C*H*₂-), 1,25(m,16H,-(C*H*₂)₈-).

### 4 Synthèse du N,N'-diformyl-N,N'-di[1-méthyl-2-(2-hydroxyéthyl)dithio-4-hydroxybut-1-ényl]-1,12-diaminododécane (TS3d)

### a) Préparation du thiosulfate de 2-hydroxyéthyle

Il est réalisé selon le §a du mode opératoire général ci-dessus, à partir du 2-chloroéthanol.
RMN ¹H (250 MHz, D₂O) : δ 3,32 (t,2H,S-C*H*₂), 3,98(t,2H,-C*H*₂-OH)

### b) Préparation de TS3d

Selon le §b du mode opératoire général ci-dessus, à partir de T3 (voir sa préparation plus loin) et du sel de Bunte obtenu précédemment, on obtient une huile.
RMN ¹H (250 MHz, CDCl₃) : δ 7,91-7,87 (2s, 2H, C*H*O), 4,61 (m, 4H, -S-CH₂-C*H*_{*2*}-OH), 3,75 (m, 4H, =C-CH₂-C*H*₂-OH), 3,33(m,4H,S-C*H*_{*2*}-), 2,87(t,4H,=C-*CH*_{*2*}), 2,78 (t, 4H,N-C*H*₂-), 1,95 (d, 6H, C*H*₃-C=), 1,45 (m, 4H, -N-CH₂-C*H*_{*2*}-), 1,20 (m, 16H, - (C*H*_{*2*})₈-).

### 5. Synthèse du N,N'-diformyl-N,N'-di[1-méthyl-2-propyldithio-4-méthoxybut-1-ényl]-1,12-diaminododécane (TS4b)

Selon le § b du mode opératoire général ci-dessus, à partir de T4 (voir sa préparation plus loin) et du thiosulfate de propyle, on obtient une huile jaunâtre.
RMN ¹H (250 MHz, CDCl₃) : δ 7,91 et 7,99 (2s, 2H, C*H*O), 3,8 (m, 4H, -C*H*₂-OH), 3,39 (m, 4H, S-C*H*₂-), 2,91 (t, 4H, =C-C*H*₂), 2,62 (t, 4H, N-C*H*₂-), 2,00 (s, 6H, C*H*₃-C=), 1,64 (m, 9H, -N-CH₂-C*H*₂- + -S-CH₂-C*H*₂-), 1,26 (m, 16H, -(C*H*₂)₈-), 0,97 (t, 6H, -S-CH₂-CH₂-C*H*₃).

### 6. Synthèse du N,N'-diformyl-N,N'-di[1-méthyl-2-propyldithio-ethényl]-1,12-diaminododécane (TS6b)

Selon le § b du mode opératoire général ci-dessus, à partir de T6 (voir sa préparation plus loin) et du thiosulfate de propxle, on obtient une huile jaunâtre.
RMN ¹H (250 MHz, CDCl₃) : δ 8,02 (s, 2H, C*H*O), 6,03 (s, 2H, =C-*H*), 3,47 (t, 4H, N-C*H*_{*2*}-), 2,69 (t, 4H, S-C*H*_{*2*}-), 1,95 (s, 6H, C*H*₃-C=), 1,72 (m, 4H, -S-CH₂-C*H*_{*2*}-), 1,59 (m, 4H, -N-CH₂-C*H*_{*2*}-) , 1,26 (m, 16H, -(C*H*₂)₈-), 0,99 (t, 6H, - S-CH₂-CH₂-C*H*_{*3*}).

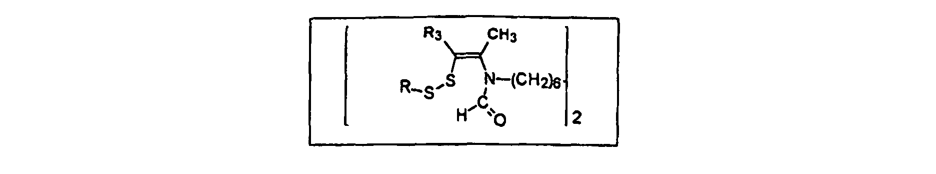

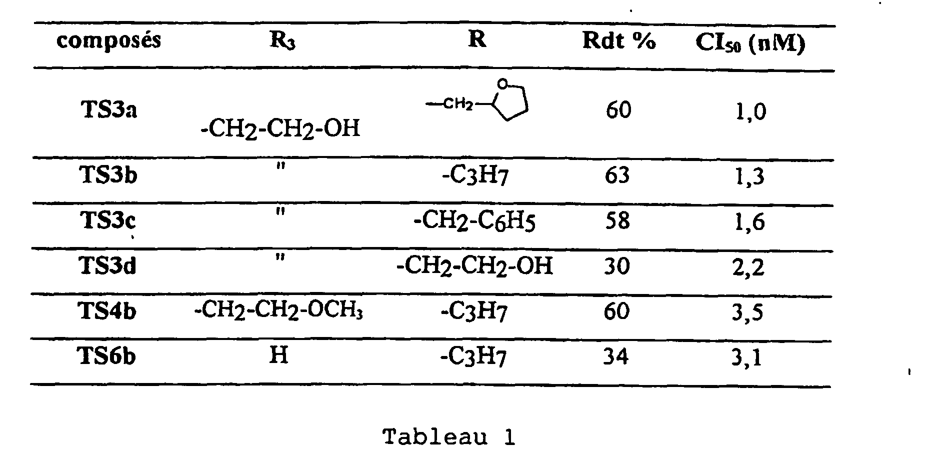

### B. Synthèse des prodrogues thioesters (TE):

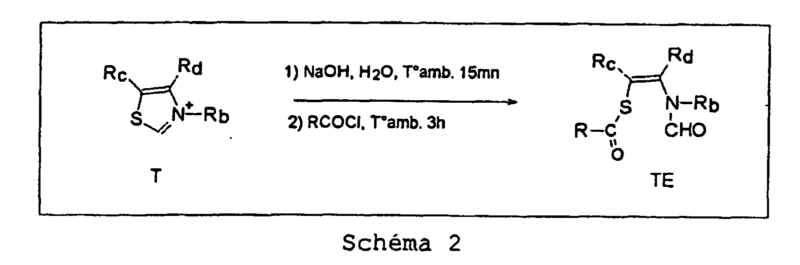

### Mode opératoire général:

3,15 mmoles du sel de thiazolium sont mis en suspension, dans 10mL d'eau et 0,75g (6 équivalents) de NaOH sont rajoutés. La solution obtenue est laissée sous agitation magnétique pendant 15 min. Ensuite, 9,6 mmoles (3 équivalents) de chlorure d'acide en solution dans 20 mL de CHCl₃ sont ajoutées goutte à goutte et le mélange réactionnel est agité à température ambiante pendant 3 heures. La phase organique est séparée, lavée avec de l'eau saturée en NaCl puis séchée sur MgSO₄ et concentrée à l'évaporateur. Le résidu obtenu est purifié sur gel de silice (éluant CH₂Cl₂/MeOH: 95/5).

### 1. Synthèse des dérivés thioesters de la série N dupliquée (composés TE4a-j, TE3 et TE8):

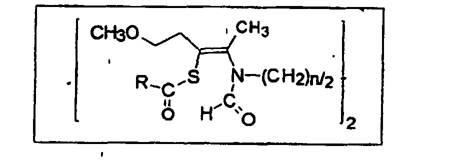

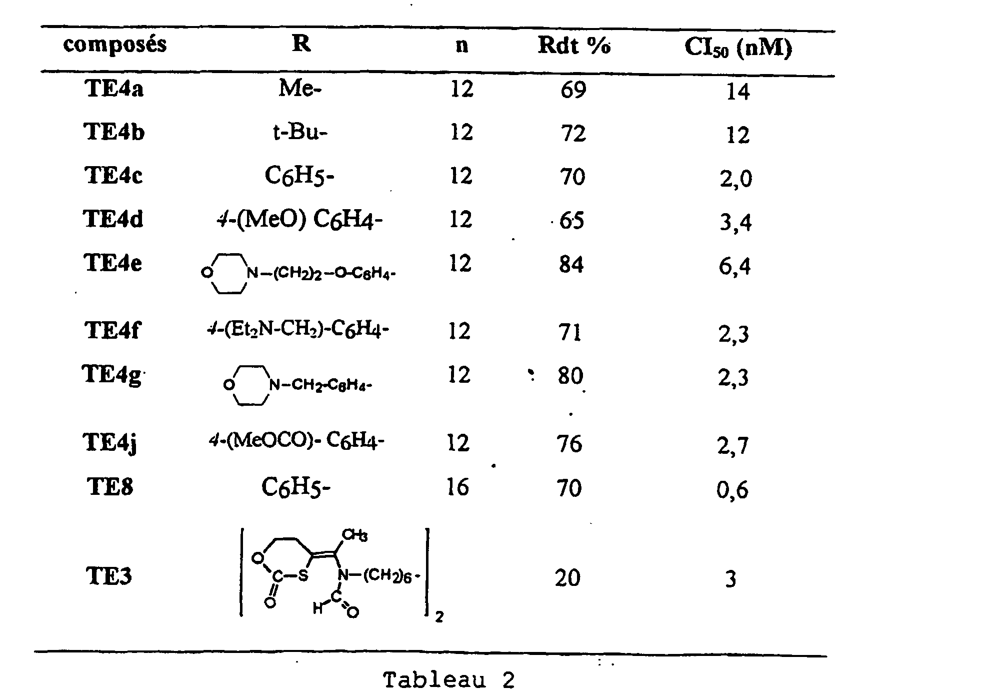

### 1.1. Synthèse du N,N'-diformyl-N,N'-di[1-méthyl-2-S-thiobenzoyl-4-méthoxybut-1-ényl]-1,12-diaminododécane (TE4c)

Selon le mode opératoire décrit ci-dessus, à partir de T4 et en utilisant le chlorure de benzoyle, on obtient une poudre blanche (Rdt = 70%)
RMN ¹H (250 MHz, CDCl₃) : δ 7,90-7,36 (m, 12H, C*H*O + Ar*H*), 3,52-3,29 (m, 8H, CH₃OC*H*₂-, N-C*H*₂-), 3,30 (s, 6H, C*H*₃O), 2,75 (t, 4H, CH₃OCH₂C*H*₂-) , 2,06 (s, 6H, C*H*₃-C=), 1,57-1,09 (m, 20H, -(C*H*₂)₁₀-).
MS ES⁺ : m/e 725 ([M+H]⁺, 100).

### 1.2. Synthèse du N,N'-diformyl-N,N'-di[1-méthyl-2-S-(p-diéthylaminométhylphénylcarboxy)-thio-4-méthoxybut-1-ényl]-1,12-diaminododécane (TE4f)

### a) Synthèse de l'acide α-diéthylamino-paratoluique

1g (1 équivalent) d'acide α-chloroparatoluique et 1,22mL (2 équivalents) de diéthylamine sont mis en solution dans 30mL d'acétonitrile. Le mélange réactionnel est porté au reflux pendant 48h. Le solvant est évaporé sous vide et le résidu est purifié par chromatographie sur colonne de gel de silice (CH₂Cl₂/MeOH 60/40 puis MeOH pur). Le produit est obtenu sous forme d'une poudre blanche après précipitation dans l'hexane. (Rdt = 63%).
RMN ¹H (250 MHz, DMSOD₆) : δ 7,84 et 7.29 (2d, 2x2H, Ar*H*) , 3,50 (s, 2H, N-C*H*₂-Ar), 2,40 (q, 4H, N(C*H*₂-CH₃)₂), 0,9 (t, 6H, N(CH₂- C*H*_{*3*})₂).
MS ES⁺ : m/e 208 ([M+H]⁺, 100).

### b) Synthèse du chlorure de l'acide α-diéthylaminoparatoluique

0,3g (1,45mmol) d'acide α-diéthylaminoparatoluique sont placés dans 10mL de CHCl₃ et 0,32mL de SOCl₂ sont rajoutés. La solution est portée au reflux pendant 12h. Le solvant est évaporé sous vide et le résidu est recristallisé dans de l'éther éthylique. On obtient 323mg de produit, sous forme de son chlorhydrate (Rdt = 85%).
MS ES⁺ : m/e 226 ([M+H]⁺, 100), 228 ([M+H]⁺, 30).

### c) Synthèse du TE4f (sous la forme de dichlorhydrate)

0,7g (0,95mmol) de T4 sont mis en suspension dans 10mL d'eau et 0,23g (5,71mmol) de NaOH sont rajoutés. La solution obtenue est laissée sous agitation pendant 15 min. Ensuite, 0,75g (2,86mmol) du chlorure de l'acide α-diéthylaminoparatoluique en solution dans 20 mL de CHCl₃ et 0,4mL (2,86mmol) de triéthylamine sont ajoutés goutte à goutte. Le mélange réactionnel est agité à température ambiante pendant 4h. La phase organique est séparée, lavée à l'eau et séchée sur MgSO₄ et concentrée au rotavapor. L'huile obtenue est purifiée par chromatographie sur colonne de gel de silice (CH₂Cl₂/MeOH 95/5 puis MeOH pur). Le chlorhydrate est obtenu par barbotage de HCl gazeux dans une solution de la base dans de l'éther à 0°C pendant 3h. Ce sel est obtenu sous forme de précipité mousseux. (Rendement = 54%).
RMN ¹H de la base libre: (250 MHz, CDCl₃) : δ 8,00-7,48 (m, 10H, C*H*O+ Ar*H*), 3,62-3,37 (m, 12H, CH₃OC*H*₂-C*H*₂-+ N-C*H*₂-Ar), 3,35 (s, 6H, C*H*₃O), 2,80 (t, 4H, N-C*H*₂-), 2,50 (q, 8H, N(C*H*₂-CH₃)₂), 2,15 (s, 6H, C*H*₃-C=), 1,50-1,12 (m, 20H, -(C*H*₂)₁₀-), 1,08 (t, 12H, N(CH₂-C*H*₃)₂).
MS ES⁺ : m/e 448,5 ([M+2H]⁺, 100).

### 1.3. Synthèse du N,N'-diformyl-N,N'-di[1-méthyl-2-S-(p-morpholino-méthylphénylcarboxy)-thio-4-méthoxybut-1-ényl]-1,12-diaminododécane (TE4g)

### a) Synthèse de l'acide α-morpholinoparatoluique

4,04g (1 équivalent) d'acide α-chloroparatoluique et 4,13g (2 équivalents) de morpholine sont mis en solution dans 150 mL de toluène. Le mélange réactionnel est porté au reflux pendant 24h. Le chorhydrate de la morpholine est éliminé par filtration à chaud sur buchner. Le produit cristallise à température ambiante dans le filtrat. Après filtration et séchage, 3,63g de produit sont obtenus sous forme d'une poudre blanche (Rdt = 70%).
RMN ¹H (250 MHz, DMSOD₆) : δ 7,84 et 7.37 (2d, 2x2H, Ar*H*), 3,53 (t, 4H, C*H*₂OC*H*₂), 3,48 (s, 2H, Ar-C*H*₂-), 2,31 (t, 4H, C*H*₂-N-C*H*₂-).
MS ES⁺ : m/e 222 ([M+H]⁺, 100).

### b) Synthèse du chlorure de l'acide α-morpholinoparatoluique

2,33g d'acide α-morpholinoparatoluique sont placés dans 30mL de CH₂Cl₂ et 3,76g de SOCl₂ sont rajoutés. La solution pas très homogène est portée au reflux pendant 48h. Le précipité blanc obtenu est filtré, lavé au CH₂Cl₂ et séché. On obtient 2,65g de produit (Rdt = 70%).
RMN ¹H (250 MHz, DMSOD₆) : δ 7,97 et 7.78 (2d, 2x2H, Ar*H*), 4,40 (s, 2H, Ar-C*H*₂-), 3,86 (m, 4H, C*H*₂OC*H*₂-), 3,18 (m, 4H, C*H*₂-N-C*H*₂-).
MS ES⁺ : m/e 240 ([M+H]⁺, 100)', 242 ([M+H]⁺, 30).

### c) Synthèse du TE4g (sous la forme du sel de dioxalate TE4go ou de ditartrate TE4gt)

1,08g de T4 sont mis en suspension dans 10mL d'eau et 0,37g (6 équivalents) de NaOH sont rajoutés. La solution obtenue est laissée sous agitation pendant 15 min. Ensuite, 1,15g (3 équivalents) du chlorhydrate du chlorure de l'acide α-morpholinoparatoluique en solution dans 20 mL de CHCl₃ et 0,42g de triéthylamine sont ajoutés goutte à goutte. Le mélange réactionnel est agité à température ambiante pendant 3h. La phase organique est extraite puis séchée sur MgSO₄ et concentrée au rotavapor. Le résidu obtenu est repris dans de l'éther et de l'eau. La phase organique est extraite, lavée 2 fois à l'eau puis séchée sur MgSO₄ et concentrée. L'huile obtenue est reprise dans un minimum d'éther et est ajoutée à une solution éthérée contenant 0,41g d'acide oxalique. Un précipité blanc se forme immédiatement (TE4go : Rendement = 80%).

Un autre échantillon d'huile (7,7 g)est mis en solution avec une solution aqueuse (1N) contenant 2 équivalents d'acide tartrique (+). La solution est évaporée à sec. Le résidu est dissous dans de l'éthanol, la solution est à nouveau évaporée. On obtient un solide mousseux (TE4gt, Pf : 82-85°C).
Caractérisation de la base libre :
RMN ¹H (250 MHz, CDCl₃) : δ 7,90-7,36 (m, 10H, CHO + Ar*H*) , 3,69 (t, 8H, C*H*₂OC*H*₂-), 3,52-3,32 (m, 12H, CH₃OC*H*₂C*H*₂- + N-C*H*₂-Ar) , 3,30 (s, 6H, C*H*₃O), 2,77 (t, 4H, N-C*H*₂-), 2,42 (t, 8H, -C*H*₂-N-C*H*₂-), 2,09 (s, 6H, C*H*₃-C=) , 1,57-1,09 (m, 20H, -(C*H*₂)₁₀-).
MS ES⁺ : m/e 462 ([M+2H]⁺, 100), m/e 923 ([M+H]⁺, 10).

### 1.4. Synthèse du N, N'-diformyl-N, N'-di[1-méthyl-2-S-(p-phtaloyl)-thio-4-méthoxybut-1-ényl]-1,12-diaminododécane (TE4j)

Selon le mode opératoire décrit ci-dessus, à partir de T4 et en utilisant le chlorure de p-méthoxycarbonylbenzoyle, on obtient une poudre blanche (Rdt = 76%).
RMN ¹H (250 MHz, CDCl₃) : δ 7,90-7,36 (m, 10H, CHO + Ar*H*) , 3,97 (s, 6H, C*H*_{*3*}OCO), 3,57-3,35 (m, 8H, CH₃OC*H*₂C*H*_{*2*}-), 3,30 (s, 6H, C*H*₃O), 2,82 (t, 4H, N-C*H*₂-), 2,13 (s, 6H, C*H*₃-C=), 1,58-1,17 (m, 20H, -(C*H*₂)₁₀-).
MS ES⁺ : m/e 421 ([M+2H]⁺⁺, 20). 841 ([M+H]⁺, 100).

### 1.5. Synthèse du N,N'-diformyl-N,N'-di[1-méthyl-2-S-thiobenzoyl-4-méthoxybut-1-ényl]-1,16-diaminohexadécane (TE8)

Selon le mode opératoire général décrit ci-dessus, à partir du diiodure de 1,16-hexadécaméthylène bis [4-méthyl-5-(2-méthoxyéthyl) thiazolium], T8, et en utilisant le chlorure de benzoyle, on obtient une huile jaunâtre (Rdt = 72%).
RMN ¹H (250 MHz, CDCl₃) : δ 7,90-7,36 (m, 12H, C*H*O + Ar*H*) , 3,50-3,32 (m, 8H, CH₃OC*H*₂-, N-C*H*₂), 3,30 (s, 6H, C*H*₃O), 2,75 (t, 4H, CH₃OCH₂C*H*₂-), 2,06 (s, 6H, C*H*₃-C=) , 1,57-1,09 (m, 28H, -(C*H*₂)₁₀-).
MS ES⁺ : m/e 781 ([M+H]⁺, 100).

### 1.6 Synthèse du N,N'-diformyl-N,N'-di[1(2-oxo-4,5-dihydro-1,3-oxathian-4-ylidène)éthyl]1,12-diaminododécane (TE3)

2,8 mmoles (2g) du sel de thiazolium T3 [dibromure de 1,12- dodécaméthylène bis [4-méthyl-5-(2-hydroxyéthyl) thiazolium] sont dissous dans 4,4 mL d'éthanol et 12,2 mmol (4,5 mL, 4 équivalents) de NaOH (10%) sont rajoutés. La solution obtenue est laissée sous agitation magnétique pendant 15 min. Ensuite, 6 mL (1, 12 g, 2 équivalents) de 4-nitrophénylchloroformate en solution dans de l'acétate d'éthyle sont ajoutés goutte à goutte et le mélange réactionnel est agité à température ambiante pendant 2 heure. On ajoute de l'acétate d'éthyle. La phase organique est lavée successivement avec de l'eau, une solution saturée d'hydrogénocarbonate de sodium, de l'eau, puis séchée sur MgSO₄ et concentrée à l'évaporateur. L'huile jaune obtenue est purifiée sur gel de silice (éluant CH₂Cl₂, puis en ajoutant 1% de MeOH, puis 2%). Rendement : 20%.
RMN ¹H (250 MHz, CDCl₃) : δ 8,2 et 7,88 (2s,2H,C*H*O) ; 4,43-4,40(t,4H,-C*H*_{*2*}OCO-) ; 3,33-3,27(t,N-C*H*₂) ; 2,79-2,77 (t,4H,CH₂-C*H*₂=) ; 1,88(s,6H,C*H*₃; 1,46-1,13(m,20H,(C*H*₂)₁₀).
MS ES⁺ : m/e 541 ([M+H]⁺, 100).

### 2. Synthèse des thioesters de la série C dupliquée

### 2.1. Composés C5 dupliqués comportant un O dans la chaîne alkyle (TE9 et TE10):

Ces prodrogues sont synthétisées selon le mode opératoire général décrit précédemment (schéma 2).
A partir du diiodure de 3,10-dioxadodécaméthylènebis[5-(1,4-diméthyl)thiazolium], T9, et du chlorure de p-méthoxybenzoyle, on obtient le 2,17-(N,N'-diformyl-N,N'-diméthyl)diamino-3,16-S-thio-p-méthoxybenzoyl-6,13-dioxaoctadéca-2,16-diène, TE9, sous forme d'une huile incolore. Rendement: 65 %.
RMN ¹H (200 MHz, CDCl₃) : δ 7,93-6,87 (m, 10H, C*H*O + Ar*H*), 3, 58 (s, 6H,2CH₃), 3, 55 (t,4H,2CH₂), 3, 37 (t, 4H,2CH₂), 2, 89 (s, 6H,2CH₃), 2,77(t,4H,2CH₂), 2,03(s,6H,2CH₃), 1,52-1,30(m,8H,4CH₂).

A partir du dibromure de 3,10-dioxadodécaméthylènebis[5-(1-benzyl,4-méthyl)-thiazolium], T10, et du chlorure de p-méthoxybenzoyle, on obtient le 2,17- (N,N'-diformyl-N,N'-dibenzyl)diamino-3,16-S-thio-p-méthoxybenzoyl-6,13-dioxaoctadéca-2,16-diène, TE10, sous forme d'une huile incolore. Rendement: 70 %.
RMN ¹H (200 MHz, CDCl₃) : δ 8,06-6,92 (m, 20H, C*H*O + Ar*H*), 4,68 (s, 4H, 2 CH₂), 3,91 (s, 6H, 2 CH₃), 3,50 (t, 4H, 2CH₂), 3,38 (t, 4H, 2CH₂), 2,75 (t, 4H, 2CH₂), 2,07 (s, 6H, 2CH₃), 1,56-1,31 (m, 8H, 4CH₂).

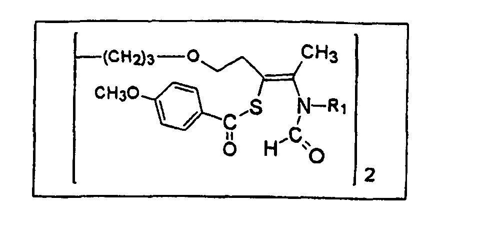

**Tableau 3**

| **composés** | **R**_{**1**} | **CI**_{**50**} **(nM)** |
|---|---|---|
| TE9 | CH₃ | 260 |
| TE10 | C₆H₅-CH₂ | 12 |

### 2.2. Composés C5 dupliqués ne comportant pas d' O dans la chaîne alkyle (TE12 et TE13):

Ces prodrogues sont synthétisées selon le mode opératoire général décrit précédemment (schéma 2).
A partir du diiodure de dodécaméthylènebis[5-(1-méthyl-4-éthoxycarbonyléthyl)-thiazolium], T12, on obtient le 3,18- (N,N'-diformyl-N,N'-diméthyl)diamino-4,17-S-thiobenzoyleicosa-3,17-diènedioate d'éthyle TE12.
Huile incolore. Rendement 70%.
RMN ¹H (200 MHz, CDCl₃) : δ 7,94-7,39 (m, 12H, C*H*O + Ar*H*), 4,14 (q, 4H, 2 OC*H*₂-), 3,45 (s, 4H, 2 C*H*_{*2*}), 2,88 (s, 6H, 2 C*H*_{*3*}), 2,44 (t, 4H, 2C*H*_{*2*}), 1,27-1,19 (m, 26H,-(C*H*₂)₁₀+ 2 C*H*_{*3*}).
A partir du dibromure de dodécaméthylènebis[5-(1-benzyl-4-éthoxycarbonyléthyl)-thiazolium], T13, on obtient le 3,18-(N,N'-diformyl-N,N'-dibenzyl)diamino-4,17-S-thiobenzoyleicosa-3,17-diènedioate d'éthyle TE13.
Huile incolore. Rendement 64%.
RMN ¹H (200 MHz, CDCl₃) : δ 8,24-7,26 (m, 22H, C*H*O + Ar*H*), 4,70 (s, 4H, 2C*H*_{*2*}-Ar), 4,23 (q, 4H, 2OC*H*₂-), 3,44 (s, 4H, 2C*H*_{*2*}), 2,51 (t, 4H, 2C*H*_{*2*}), 1,52-1,29 (m, 26H,-(C*H*_{*2*})₁₀+ 2C*H*_{*3*}).

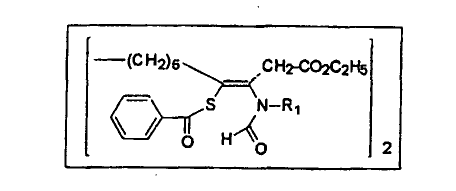

**Tableau 4**

| **composés** | **R**_{**1**} | **CI**_{**50**} **(nM)** |
|---|---|---|
| **TE12** | CH₃- | 16 |
| **TE13** | C₆H₅-CH₂- | 650 |

### 2.3. Composés C4 dupliqués ne comportant pas d' O dans la chaîne alkyle.

Ces prodrogues sont synthétisées selon le mode opératoire général décrit précédemment (schéma 2).
A partir du diiodure de dodécaméthylènebis[4-(1-méthyl)-thiazolium], T15, on obtient le 2,15-(N,N'-diformyl-N,N'-diméthyl)diamino-1,16-S-thiobenzoyl-hexadéca-1,15-diène, TE15.
Huile incolore. Rendement 70%.
RMN ¹H (200 MHz, CDCl₃) : δ 7,94-7,39 (m, 12H, C*H*O + Ar*H*) , 5,7 (2H, =C*H*), 2,88 (s, 6H, 2 N-C*H*_{*3*}), 2,48 (t, 4H, 2 =C-C*H*_{*2*}), 1,27-1,19 (m, 20H,-(C*H*₂)₁₀).
A partir du dibromure de dodécaméthylènebis[4-(1-benzyl)-thiazolium], T16, on obtient le 2,15-(N,N'-diformyl-N,N'-dibenzyl)diamino-1,16-S-thiobenzoyl-hexadéca-1,15-diène, TE16.
Huile incolore. Rendement 64%.
RMN ¹H (200 MHz, CDCl₃) : δ 8,24-7,56 (m, 22H, C*H*O + Ar*H*), 5, 7 (2H, =C*H*), 4,37 (s, 4H, 2C*H*₂-Ar), 2,51 (t, 4H, 2 =C-C*H*₂), 1,52-1,29 (m, 20H).

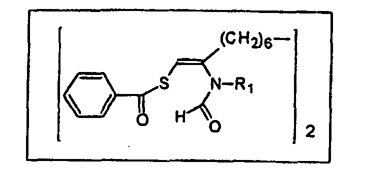

**Tableau 5**

| **composés** | **R**_{**1**} | **CI**_{**50**} **(nM)** |
|---|---|---|
| **TE15** | CH₃- | 7 nM |
| **TE16** | C₆H₅-CH₂- | 12 nM |
| | | |

### II. SYNTHESE DES SELS DE THIAZOLIUM

### A. Synthèse des composés de la série N dupliquée (composés T3, T4, T6 et T8):

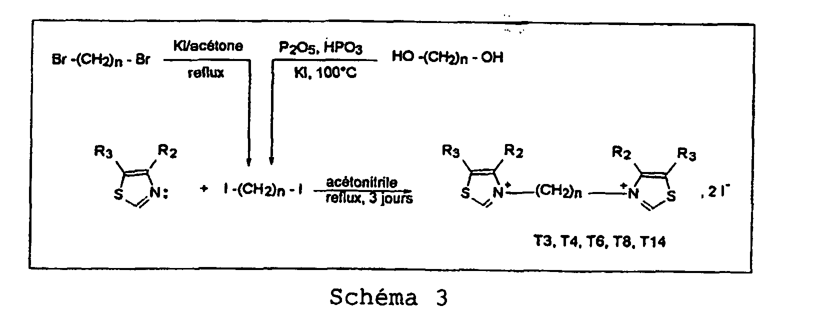

### Mode opératoire général de synthèse des dihalogénures de α,ω-polyméthylène bis thiazolium:

Le thiazole convenablement substitué (11,5 mmol) est dissous dans 30mL d'acétonitrile. 3,8 mmol du diiodure (ou dibromure) d'alkyle sont ajoutés et le mélange réactionnel est porté au reflux pendant 3 jours. La solution est concentrée à l'évaporateur rotatif et le résidu huileux obtenu est repris dans de l'eau et de l'éther. La phase aqueuse est lavée à l'éther puis concentrée sous pression réduite. Le produit est ensuite cristallisé dans l'isopropanol.
Le 1,12-diiodododécane est synthètisé de la façon suivante:
On mélange 10,22g (1 équivalent) de 1,12-dibromododécane avec 14,26g (3 équivalents) d'iodure de sodium dans 150 mL d'acétone. Après 15 minutes d'agitation à température ambiante, la solution est portée au reflux pendant 3 heures.
L'acétone est ensuite évaporée au rotavapor, le résidu est repris dans de l'éther éthylique et de l'eau et le produit est extrait trois fois à l'éther. Les phases étherées sont regroupées et sont séchées sur sulfate de magnésium. Le solide blanc obtenu est ensuite recristallisé dans le méthanol (Pf 42-43°C). Rendement= 95%.

Le 1,16-diiodohexadécane est obtenu à partir de l'hexadécane-1,16-diol, par addition de 5g de ce diol et 19g d'iodure de potassium à une solution de 2,5g d'anhydride phosphorique et 5,2mL d'acide phosphorique 85%. Le mélange est chauffé à 100°C pendant 5h. Une huile dense se forme, et le mélange est versé dans 50mL d'eau. La phase organique est séparée, et la phase aqueuse extraite à l'éther. Les phases organiques sont décolorées par agitation avec 50mL d'une solution de thiosulfate de sodium 10%. L'éther est évaporé. L'huile obtenue est cristallisée dans le méthanol (Pf= 52°C). Rendement: 82%.

### 1- Synthèse du dibromure de 1,12- dodécaméthylène bis [4-méthyl-5-(2-hydroxyéthyl) thiazolium] (T3)

Selon le mode opératoire décrit ci-dessus, à partir du 4-méthyl-5-[2-hydroxyéthyl] thiazole et du 1,12-dibromododécane, on obtient une poudre blanche hygroscopique (Rdt = 75%)
RMN ¹H (250 MHz, DMSO D₆) : δ 10,08 (s, 2H, S-C*H*=), 4,45 (t, 4H, ⁺N-C*H*₂-), 3,62 (t, 4H, HOC*H*₂CH₂-), 3,02 (t, 4H, HOCH₂C*H*_{*2*}-), 2,50 (s, 6H, C*H*₃-C=), 1,77 (m, 4H, ⁺N-CH₂C*H*₂-), 1,60-1,25 (m, 16H, -(C*H*₂)₈-).
MS ES⁺ : m/e 227 (M⁺⁺, 100), m/e 533-535 (M⁺⁺Br⁻, 10)

### 2- Synthèse du diiodure de 1,12-dodécaméthylène bis[4-méthyl-5-(2-méthoxyéthyl)thiazolium] (T4)

Selon le mode opératoire général décrit ci-dessus, à partir du 4-méthyl-5-(2-méthoxyéthyl) thiazole et du 1,12-diiodododécane, on obtient une poudre blanche hygroscopique (Rdt = 70%)
RMN ¹H (250 MHz, CDCl₃) : δ 10,92 (s, 2H, S-C*H*=), 4,66 (t, 4H, ⁺N-C*H*₂-), 3, 60 (t, 4H, CH₃OC*H*₂CH₂-), 3,35 (s, 6H, C*H*₃O), 3,07 (t, 4H, CH₃OCH₂C*H*_{*2*}-), 2, 52 (s, 6H, C*H*₃-C=), 1,92 (m, 4H, ⁺N-CH₂C*H*₂-), 1,57-1,25 (m, 16H, -(C*H*₂)₈-).
MS ES⁺ : m/e 241 (M⁺⁺, 100 ), m/e 609 (M⁺⁺I⁻, 5)

Le 4-méthyl-5-[2-méthoxyéthyl] thiazole est synthétisé selon le procédé ci-dessous:
10,20mL de 4-méthyl-5-[2-hydroxyéthyl] thiazole sont dissous dans 180 mL de DMSO anhydre et 19g de potasse en poudre sont ajoutés. Après 5 mn d'agitation, 5,30mL d'iodure de méthyle sont introduits. Le mélange réactionnel est agité pendant 30 mn à température ambiante sous atmosphère inerte. A l'issue de la réaction (suivie par CCM), le mélange est versé dans 100 mL d'eau et on extrait 3 fois à l'éther. La phase organique est ensuite lavée à l'eau, puis à l'eau saturée en NaCl et enfin, séchée sur sulfate de sodium. Le produit obtenu est purifié sur gel de silice en éluant avec un mélange AcOEt/hexane (1/3). On obtient une huile jaune (Rdt = 60%)
RMN ¹H (250 MHz, CDCl₃) : δ 2,39 (s, 3H, C*H*_{*3*}C=) , 3, 00 (t, 2H, -C*H*_{*2*}C=) , 3,36 (s, 3H, C*H*_{*3*}O), 3,55 (t, 2H, O-C*H*_{*2*}-),8,56 ( s, 1H, S-C*H*=).

### 3- Synthèse du diiodure de 1,12-dodécaméthylènebis(4-méthylthiazolium) (T6)

Selon le mode opératoire général décrit ci-dessus, à partir du 4-méthylthiazole et du 1,12-diiodododécane, on, obtient une poudre blanche (Rdt = 50%)
RMN ¹H (250 MHz, DMSO D6) : δ 10,11 (s, 2H, S-C*H*=), 8,02 (s, 2H, S-C*H*=), 4,42 (t, 4H, ⁺N-C*H*₂-), 2,55 (s, 6H, C*H*₃-C=) , 1, 80 (m, 4H, ⁺N-CH₂C*H*₂-), 1,25 (m, 16H, -(C*H*₂)₈-).
MS ES⁺ : m/e 183 (M⁺⁺, 100 ), m/e 493 (M⁺⁺I⁻, 5)

### 4- Synthèse du diiodure de 1,16-hexadécaméthylènebis[4-méthyl-5-(2-méthoxyéthyl)thiazolium] (T8)

Selon le mode opératoire général décrit ci-dessus, à partir du 4-méthyl-5-[2-méthoxyéthyl] thiazole et du 1,16-diiodohexadécane, on obtient une poudre blanche (Rdt = 80%). Pf: 210°C.
RMN ¹H (250 MHz, CDCl₃) : δ 10,92 (s, 2H, S-C*H*=) , 4,66 (t, 4H, ⁺N-C*H*_{*2*}-), 3,60 (t, 4H, CH₃OC*H*_{*2*}CH₂-), 3,35 (s, 6H, C*H*_{*3*}O), 3,07 (t, 4H, CH₃OCH₂C*H*_{*2*}-), 2,52 (s, 6H, C*H*₃-C=), 1,92 (m, 4H, ⁺N-CH₂C*H*_{*2*}-), 1,57-1,25 (m, 24H, -(C*H*₂)₈-).
MS ES⁺ : m/e 269 (M⁺⁺, 100 ) , m/e 665 (M⁺⁺I⁻, 10)

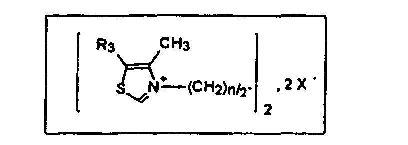

**Tableau 6**

| **composés** | **R**_{**3**} | **n** | **X** | **CI**_{**50**} **(nM)** |
|---|---|---|---|---|
| **T3** | -CH₂-CH₂-OH | 12 | Br | 2,25 |
| **T4** | -CH₂-CH₂-OCH₃ | " | " | 0,65 |
| **T6** | H | " | I | 3 |
| **T8** | -CH₂-CH₂-OCH₃ | 16 | " | 1,1 |

### B. Synthèse des composés de la série C dupliquée (T9, T10, T12 et T13) :

### 1- Synthèse des composés dupliqués sur le carbone C5 du cycle thiazole.

### 1-1- Synthèse des composés comportant un O dans la chaîne alkyle (T9, T10):

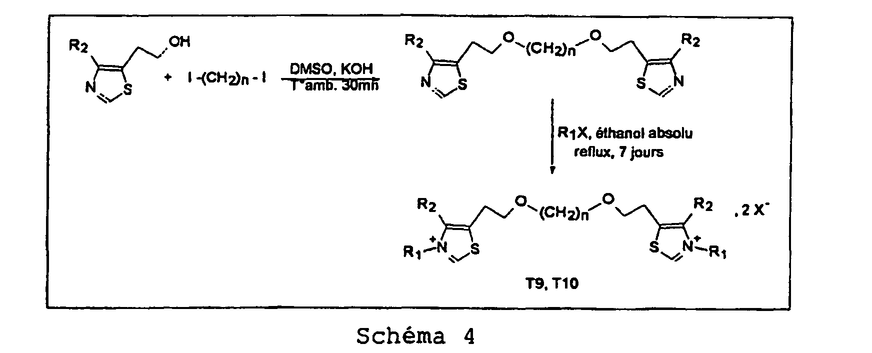

### Mode opératoire général de synthèse des diiodures de 3,10-dioxadodécaméthylènebis[5-(1-alkyl-4-méthyl)thiazolium]: T9, T10) (Schéma 4).

1^{ère} étape: Dissoudre le 4-méthyl-5-hydroxyéthylthiazole (20,9 mmol) dans du DMSO anhydre (50 ml). Ajouter l'hydroxyde de potassium (83,6 mmol) et laisser agiter pendant 10mn. Ajouter le dérivé diiodé (6,9 mmol) et laisser agiter à température ambiante pendant 30mn. Ajouter de l'eau et extraire 3 fois à l'éther. Laver la phase éthérée avec de l'eau, la sécher sur sulfate de sodium. Purifier par chromatographie sur gel de silice en éluant avec AcOEt-Hexane (1-3).

2^{ème} étape: Alkylation du bis-thiazole :
Dissoudre le bis-thiazole (1 mmol) dans de l'éthanol absolu (40 ml). Ajouter le dérivé halogéné souhaité (2 mmol) et chauffer le mélange sous reflux pendant environ une semaine. Evaporer l'éthanol et recristalliser dans un mélange iPrOH-(iPr)₂O.

### a) Synthèse du 1,6-bis[2-(4-méthylthiazol-5-yl)éthoxyhexane:

Selon le mode opératoire général (1^{ère} étape) décrit ci-dessus, à partir du 4-méthyl-5-hydroxyéthylthiazole et du 1,6-diiodohexane, on obtient une huile incolore(Rdt = 60%) 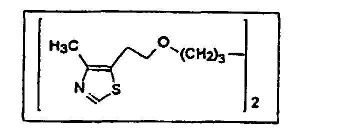 RMN ¹H (200 MHz, CDCl₃) : d 8,51 (s, 2H, CH), 3,54 (t, 4H, -CH₂-), 3,39 (t, 4H, -CH₂), 2,96 (t, 4H, -CH₂-), 2,32 (s, 6H, CH₃), 1,57-1,28 (m, 8H, -CH₂-CH₂-).

b) Synthèse du diiodure de 3,10-dioxadodécaméthylènebis[5-(1,4-diméthyl)thiazolium] (T9) Selon le mode opératoire général (2^{ème} étape) décrit ci-dessus, à partir du produit précédemment obtenu et de l'iodure de méthyle, on obtient un solide blanc hygroscopique(Rdt = 60%)
RMN ¹H (200 MHz, CDCl₃) : d 10,99 (s, 2H, S-C*H*=), 4,33 (s, 6H, 2N⁺C*H*_{*3*}), 3,71 (t, 4H, 2C*H*_{*2*}-O), 3,52 (t, 4H, 2*O*-C*H*_{*2*}), 3,03 (t, 4H, 2C*H*_{*2*}), 2,51 (s, 6H, 2 =C-C*H*_{*3*}), 1,65-1,47 (m, 8H, 4-C*H*_{*2*}-).

### c) Synthèse du dibromure de 3,10-dioxadodécaméthylènebis[5-(1-benzyl,4-méthyl)-thiazolium] (T10)

Selon le mode opératoire général (2^{ème} étape) décrit ci-dessus, à partir du produit obtenu dans la 1^{ère} étape et du bromure de benzyle, on obtient un solide blanc hygroscopique(Rdt = 74%)
RMN ¹H (200 MHz, CDCl₃) : d 11,45 (s, 2H, S-C*H*=), 7,36-7,28 (m, 10H, 2Ar*H*), 3,65 (t, 4H, 2C*H*_{*2*}-O), 3,44 (t, 4H, 2O-C*H*_{*2*}), 3,03 (t, 4H, 2C*H*_{*2*}), 2,51 (s, 6H, 2 =C-C*H*_{*3*}), 1,57-1,34 (m, 8H, 4-C*H*_{*2*}-).

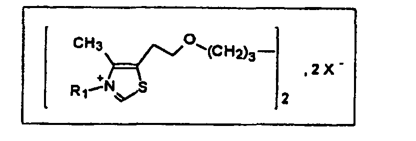

**Tableau 7**

| **composés** | **R**_{**1**} | **X** | **CI**_{**50**} **(nM)** |
|---|---|---|---|
| **T9** | CH₃- | I | 70 |
| **T10** | C₆H₅-CH₂- | Br | 2,5 |

### 1-2- Composés ne comportant pas d' O dans la chaîne alkyle (T12, T13)

Cette synthèse se fait en 4 étapes selon le schéma 5.

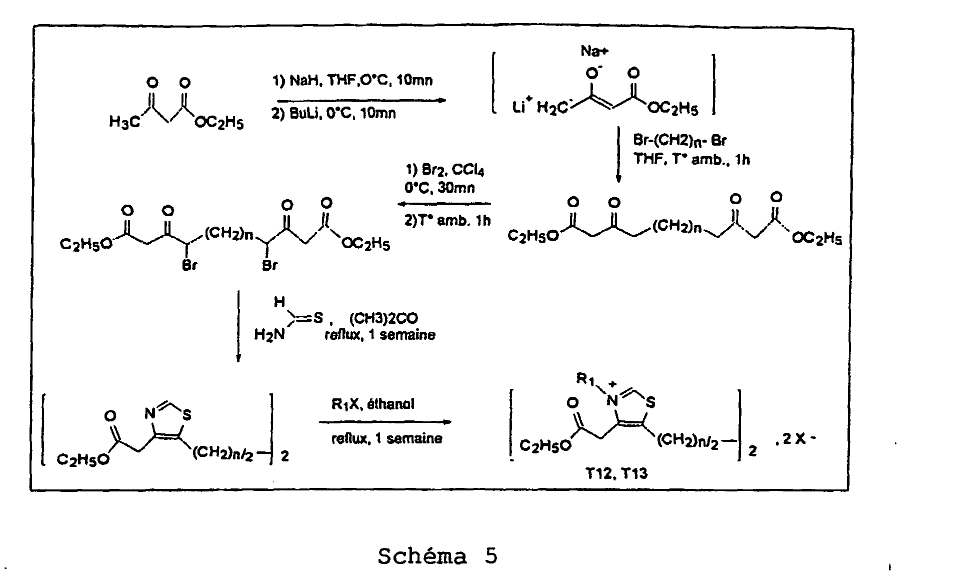

### a) Synthèse du 3,18-dioxoeicosanedioate d'éthyle:

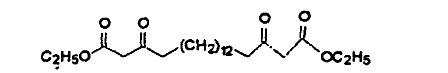

Dans un bicol sous argon, mettre en suspension NaH (43,7 mmol) dans du THF anhydre (100 ml). Refroidir le milieu réactionnel dans un bain de glace et ajouter goutte à goutte l'acétoacétate d'éthyle (39,7 mmol). Laisser agiter pendant 10mn à 0°C. Ajouter goutte à goutte du n-BuLi (1,56 M; 43,7 mmol). Laisser agiter 10mn supplémentaires à température ambiante avant de passer à l'alkylation.

A la solution précédente, on ajoute goutte à goutte le dibromododécane (15,9 mmol) en solution dans 20 ml de THF anhydre. On laisse revenir le milieu réactionnel à température ambiante et on continue l'agitation pendant 1h. On ajoute de l'eau et on extrait à l'éther (3 fois). La phase organique est lavée avec une solution NaCl saturée, séchée sur sulfate de sodium, filtrée et évaporée à sec. On purifie le produit par chromatographie sur gel de silice en éluant avec de l'AcOEt-Hexane (1-1). On obtient solide blanc (Rdt = 65 %); Pf = 60°C.
RMN ¹H (200 MHz, CDCl₃) : d 4,16 (q, 4H, 2CH₂), 3,41 (s, 4H, 2CH₂), 2,50 (t, 4H, 2CH₂), 1,56-121 (m, 30H).

### b) Synthèse du 4,17-dibromo-3,18-dioxoeicosanedioate d'éthyle:

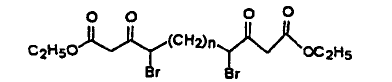

Le composé précédent (3,8 mmol) est dissous dans 20 ml de CCl₄. La solution est refroidie à 0°C, et on ajoute goutte à goutte du brome (76 mmol), on laisse agiter à la même température pendant 30mn, puis température ambiante pendant 1h. On evapore le solvant. Reprendre Le résidu est dissous dans de l'eau, et on extrait à l'acétate d'éthyle, la phase organique est séchée sur sulfate de sodium, filtrée et évaporée à sec. On purifie par chromatographie sur gel de silice en éluant avec l'AcOEt-Hexane (1-1). On obtient une huile jaunâtre (Rdt = 64 %). RMN ¹H (200 MHz, CDCl₃) : d 4,42 (m, 6H, 2CH₂+CH), 3,78-3,48 (m, 4H, 2CH₂), 1,94-1,14 (m, 30H).

### c) Synthèse du dodécaméthylènebis[5-(4-éthoxycarbonyléthyl)thiazole]:

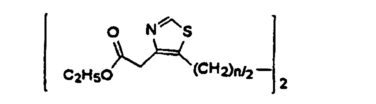

A une solution de 11,7 mmol de bis-bromoacétoacétate d'éthyle dans 5 ml d'acétone on ajoute.du thioformamide (23,4 mmol) en solution dans 5 ml d'acétone et on agite à 40°C pendant une semaine. Le solvant est évaporé, et le résidu. dissous dans l'eau. La solution est extraite à l'AcOEt. On purifie par chromatographie (AcOEt-Hex : 1/1). On obtient solide blanc (Rdt = 50 %); Pf = 112-114°C.
RMN ¹H (200 MHz, CDCl₃) : d 8,54 (s, 2H, 2CH), 4,17 (q, 4H, 2CH₂), 3,72 (s, 4H, 2CH₂), 2,72 (t, 4H, 2CH₂), 1,56-121 (m, 30H).

### d) Alkylation du dodécaméthylènebis[5-(4-éthoxycarbonyléthyl)thiazole]:

Le composé précédent conduit par alkylation sur l'atome d'azote (selon le procédé habituel décrit pour T3 ou T4) soit à T12 en utilisant l'iodométhane, soit à T13 en utilisant le bromure de benzyle.

### Diiodure de dodécaméthylènebis[5-(1-méthyl-4-éthoxycarbonyléthyl)-thiazolium] (T12):

Solide jaune Pf = 112°C; Rdt = 55 %
RMN ¹H (200 MHz, CDCl₃) : d 10,91 (d, 2H, 2CH) , 4,42 (6H, N-C*H*₃), 4,25-4,16 (m, 8H, 2CH₂+2CH₂), 2,91 (m, 4H, 2CH₂), 1,62-1,26 (m, 26H).

### Dibromure de dodécaméthylènebis[5-(1-méthyl-4-éthoxycarbonyléthyl)-thiazolium] (T13):

Huile jaune; Rdt = 50 %
RMN ¹H (200 MHz, CDCl₃) : d 11,26 (d, 2H, 2CH), 7,36-7,26 (m, 10H, ArH), 6,07(s, 4H, 2CH₂), 4,02-3,92 (m, 12H, 6CH₂), 2,91 (m, 4H, 2CH₂), 1,62-1,26 (m, 26H).

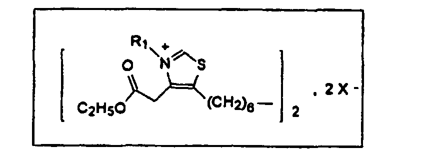

**Tableau 8**

| **composés** | **R**_{**1**} | **X** | **CI**_{**50**} **(nM)** |
|---|---|---|---|
| **T12** | CH₃- | I | 13 |
| **T13** | C₆H₅-CH₂- | Br | 250 |

### 2- Synthèse des composés dupliqués sur le carbone C4 du cycle thiazole (T15 et T16).

Cette synthèse se fait en 5 étapes selon le schéma 6.

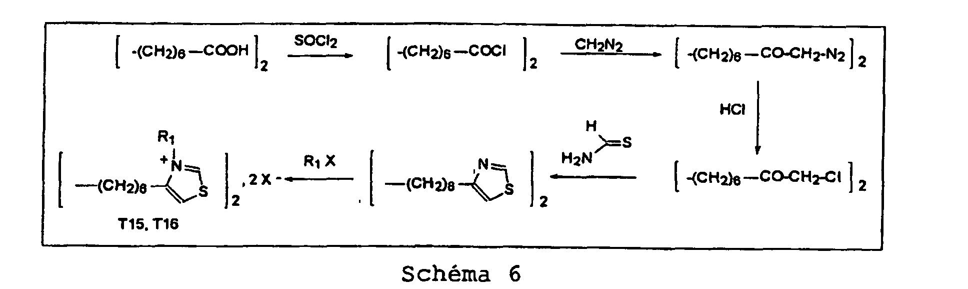

L'acide tétradécanedioïque est converti en son chlorure (Jayasuriya et coll.; J.Amer.Chem.Soc.; 112;15; 1990; 5844-5850). Celui-ci est traité par le diazométhane pour conduire au 1,16-bis-diazo-hexadécane-2,15-dione (Canonica et coll.; Atti Accad.Naz.Lincei Cl.Sci.Fis.Mat.Nat.Rend.; 8,10; 1951; 479-484), qui est traité par HCl et donne la 1,16-dichlorohexadécane-2,15-dione (même référence). Ce dernier composé est ensuite traité par la thioformamide dans les mêmes conditions que pour la synthèse du dodécaméthylènebis[5-(4-éthoxycarbonyléthyl)thiazole] (schéma 5, 3^{ème} étape) pour donner le dodécaméthylènebis(4-thiazole).
Celui-ci conduit par alkylation sur l'atome d'azote (selon le procédé habituel décrit pour T3 ou T4) soit au diiodure de dodécaméthylènebis[4-(1-méthyl)-thiazolium], T15, en utilisant l'iodométhane, soit au dibromure de dodécaméthylènebis[4-(1-benzyl)-thiazolium], T16, en utilisant le bromure de benzyle.

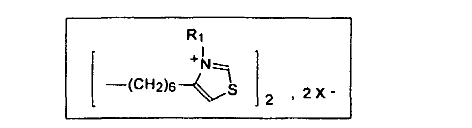

**Tableau 9**

| **composés** | **R**_{**1**} | **X** | **CI**_{**50**} **(nM)** |
|---|---|---|---|
| **T15** | CH₃- | I | 4 |
| **T16** | C₆H₅-CH₂- | Br | 10 |

### III. SYNTHESE DES HALOALKYLAMINES

### 1) Dichlorhydrate du N,N'-diméthyl-N,N'-(5-chloropentyl)-1,16-hexadecanediamine (P1).

### a) 5-Méthylamino-1-pentanol.

10,8g (0.088 mole) de 5-chloro-1-pentanol sont ajoutés à 45ml d'une solution 8M de MeNH₂ (0,36 mole) dans le MeOH. Le mélange réactionnel est chauffé à 100°C dans un autoclave pendant 48h. Le résidu obtenu après évaporation du MeOH est distillé sous pression réduite pour donner 6,2g (65%) d'aminoalcool.
**RMN** ^{**1**}**H** (CDCl₃) δ 3,60 (t, 2H, C*H*_{*2*}OH), 2,56 (t, 2H, C*H*_{*2*}NH), 2,40 (s, 3H, C*H*_{*3*}NH), 2,66-1,32 (m, 6H, (C*H*_{*2*})₃).
**SM** (Electrospray, mode positif) m/z 118 (M+H]⁺, 100).

### b) N,N'-diméthyl-N,N'-(5-hydroxypentyl)-1,16-hexadécanediamine.

0,57g (0,0048 mole) de diisopropyléthylamine et 0,58g (0,0045 mole) de 5-Méthylamino-1-pentanol sont ajoutés à 1,08g (0,0022 mole) de diiodohexadecane dissous dans 50mL d'éthanol. Le mélange réactionnel est porté au reflux pendant 48 h, l'éthanol est ensuite éliminé sous pression réduite. L'analyse par CCM du résidu montre la formation du produit attendu et indique la présence d'une petite quantité d'un composé très polaire identifié par spectrométrie de masse comme étant le sel d'ammonium quaternaire présenté sur la figure ci-dessous.

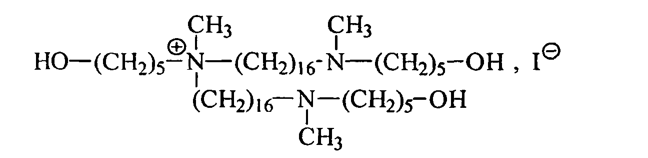

Le résidu est partiellement dissous dans l'eau et le N,N'-diméthyl-N,N'-(5-hydroxypentyl)-1,16-hexadécane-diamine est extrait dans l'éther avec K₂CO₃, laissant le contaminant polaire dans l'eau. Les phases éthérées sont séchées sur MgSO₄, filtrées et concentrées sous pression réduite.
**RMN** ^{**1**}**H** (CDCl₃) δ 3,60 (t, 4H, C*H*_{*2*}OH), 2,65-2,40 (m, 8H, C*H*_{*2*}-NH-C*H*₂), ), 2, 37 (s, 6H, C*H*_{*3*}N), 1,60-1,21 (m, 40H, 2(C*H*₂)₃ + (C*H*_{*2*})₁₄).

### c) Dichlorhydrate du N,N'-diméthyl-N,N'-(5-chloropentyl)-1,16-hexadecanediamine (P1).

Le résidu obtenu ci-dessus est dissous dans 10 ml de CH₂Cl₂ et 1,7ml de chlorure de thionyle sont ajoutés. Le mélange réactionnel est porté au reflux pendant 5h après quoi tous les produits volatiles sont éliminés sous pression réduite. Le résidu est trituré dans l'éther jusqu'à ce qu'un précipité apparaisse. Le précipité est filtré puis recristallisé dans un mélange éthanol-éther pour donner 0,408g (32%) de Dichlorhydrate de N,N'-diméthyl-N,N'-(5-chloropentyl)-1,16-hexadécanediamine.
**RMN** ^{**1**}**H** (CDCl₃) δ 3,48 (t, 4H, C*H*_{*2*}Cl), 3,00-2,80 (m, 8H, C*H*_{*2*}-NH⁺(CH₃)-C*H*_{*2*})), 2,70 (d, 6H, C*H*_{*3*}NH⁺), 1,86-1,19 (m, 40H, 2(C*H*_{*2*})₃ + (C*H*_{*2*})₁₄).
**SM** (Electrospray, mode positif) m/z 247 (M⁺⁺, 100), m/z 529/531 (M⁺⁺Cl⁻, 20).

### 2) Dichlorhydrate du N,N'-diméthyl-N,N'-(4-chloropentyl)-1,16-hexadécanediamine (P2).

### a) 5-Méthylamino-2-pentanol.

10, 59g (0,10 mole) de γ-valérolactone sont ajoutés à 60ml d'une solution 8M de MeNH₂ (0,48 mole) dans le MeOH. Le mélange réactionnel est chauffé à 100°C dans un autoclave pendant 48h. Le résidu obtenu après évaporation de l'excès de MeNH₂ sous pression réduite est dissous dans 20ml de THF et est ajouté à une solution de 6,26g (0,17mol) de LiAlH₄ dans 80 ml de THF durant 1h pour obtenir un léger reflux. Le reflux est maintenu pendant 48h, une solution de soude 5M est ensuite ajoutée goutte à goutte jusqu'à obtention d'une suspension blanchâtre. Le mélange réactionnel est extrait à l'éther, les phases éthérées sont séchées sur MgSO₄ anhydre et le solvant est éliminé sous pression réduite. Le résidu obtenu est distillé sous pression réduite pour donner 6,27g (63%) de 5-methylamino-2-pentanol.
**RMN** ^{**1**}**H** (CDCl₃) δ 3,72 (m, 1H, C*H*OH) , 2,76-2,48 (m, 2H, C*H*_{*2*}NH), 2,47 (s, 3H, C*H*_{*3*}NH), 2,66-1,32 (m, 6H, (C*H*_{*2*})₃).
**SM** (Electrospray, mode positif) m/z 118 (M+H┐⁺, 100).

### b) N,N'-diméthyl-N,N'-(4-hydroxypentyl)-1,16-hexadécanediamine.

0,59g (4,6 mmole) de diisopropyléthylamine et 0,61g (5,8 mmole) de 5-methylamino-2-pentanol sont ajoutés à 1,10g (2,3 mmole) de diiodohexadécane dissous dans 50mL d'éthanol. Le mélange réactionnel est porté au reflux pendant 48 h, l'éthanol est ensuite éliminé sous pression réduite. L'analyse par CCM du résidu montre la formation du produit attendu et indique la présence d'une petite quantité d'un composé très polaire identifié par spectrométrie de masse comme étant le sel d'ammonium quaternaire présenté sur la figure ci-dessous

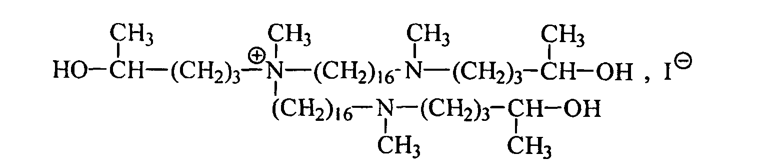

Le résidu est partiellement dissous dans l'eau et la N,N'-diméthyl-N,N'-(4-hydroxypentyl)-1,16-hexadécanediamine est extrait dans l'éther avec K₂CO₃, laissant le contaminant polaire dans l'eau. Les phases éthérées sont séchées sur MgSO₄, filtrées et concentrées sous pression réduite.
**RMN** ^{**1**}**H** (CDCl₃) δ 3,70-3,58 (m, 2H, C*H*OH), 2,40-2,24 (m, 8H, C*H*_{*2*}-NH⁺(CH₃)-C*H*_{*2*}), 2,16 (s, 6H, C*H*_{*3*}NH⁺), 1,66-1,10 (m, 42H, 2(C*H*_{*2*})₂ + 2(C*H*_{*3*}CH) + (C*H*_{*2*})₁₄).

### c) Dichlorhydrate du N,N'-diméthyl-N,N'-(4-chloropentyl)-1,16-hexadécanediamine (P2).

Le résidu obtenu ci-dessus est dissous dans 10 ml de CH₂Cl₂ et 2mL de chlorure de thionyle sont ajoutés. Le mélange réactionnel est porté au reflux pendant 5h après quoi tous les produits volatiles sont éliminés sous pression réduite. Le résidu est trituré dans l'éther jusqu'à ce qu'un précipité apparaisse. Le précipité est filtré puis recristallisé dans un mélange éthanol-éther pour donner 0,415g (32%) de dichlorhydrate de N,N'-diméthyl-N,N'-(4-chloropentyl)-1,16-hexadécanediamine.
**RMN** ^{**1**}**H** (CDCl₃) δ 4,00 (m, 2H, C*H*Cl), 3,00-2,87 (m, 8H, C*H*_{*2*}-NH⁺(CH₃)-C*H*_{*2*}),), 2,72 (d, 6H, C*H*_{*3*}NH⁺), 1,86-1,19 (m, 42H, 2(C*H*_{*2*})₂ + 2(C*H*_{*3*}CH) | (C*H*_{*2*})₁₄).
**SM** (Electrospray, mode positif) m/z 247 (M⁺⁺, 100), m/z 529/531 (M⁺⁺Cl⁻, 20).

### Etude des activités pharmacologiques de précurseurs selon l'invention.

### A. Activité antipaludique contre P. falciparum in vitro

On rapporte dans les tableaux 10 et 11 ci-après les résultats des valeurs de CI₅₀ en nM pour les prodrogues de type disulfure (tableau 10) et celles de type thioester (tableau 11) selon l'invention, ainsi que pour les drogues correspondantes.

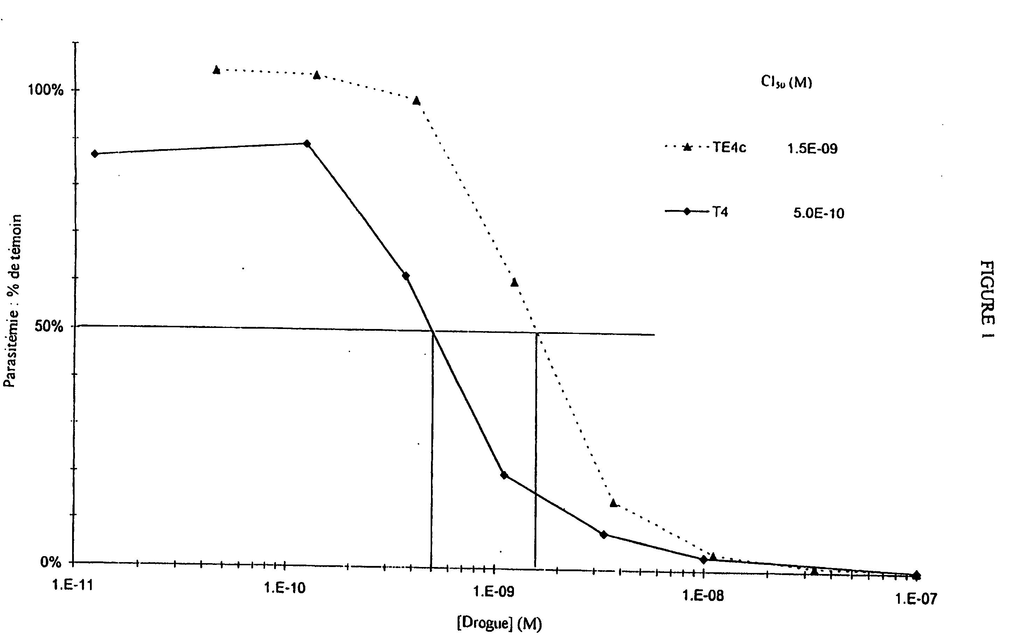

Les mesures de CI₅₀ sont déterminées vis-à-vis de P. *falciparum* selon la méthode de Desjardins dans laquelle l'incorporation d'[³H] hypoxanthine (fig.1) dans les acides nucléiques sert d'index de la viabilité cellulaire. Dans chacun des cas, des contrôles en microscopie optique sont réalisés.

**Tableau 10**

| **Prodrogue TS (Ra=S-R)** | | | | | **Drogue T** | |
|---|---|---|---|---|---|---|
| R | Z | R₃ | nom | IC₅₀ (nM) | nom | IC₅₀ (nM) |
| TEF-CH₂- | | | TS3a | 1 | | |
| C₃H₇- | -(CH₂)₁₂- | HO-CH₂-CH₂- | TS3b | 1,3 | T3 | 2,25 |
| C₆H₅-CH₂- | | - | TS3c | 1,6 | | |
| HO-CH₂-CH₂- | | | TS3d | 2,5 | | |
| C₃H₇- | | | TS4b | 3,5 | | |
| | " | CH₃O-CH₂-CH₂- | | | T4 | 0,65 |
| Et₂N(CH₂)₂- | | | TS4c | 2,8 | | |
| C₃H₇- | " | C₆H₅-COO-(CH₂)₂- | TS5 | 22 | / | |
| C₃H₇- | " | H- | TS6b | 3,1 | T6 | 3 |

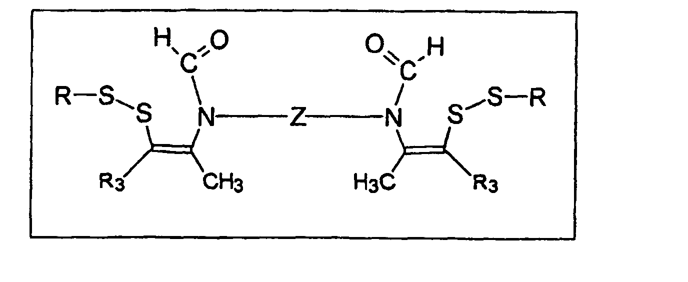

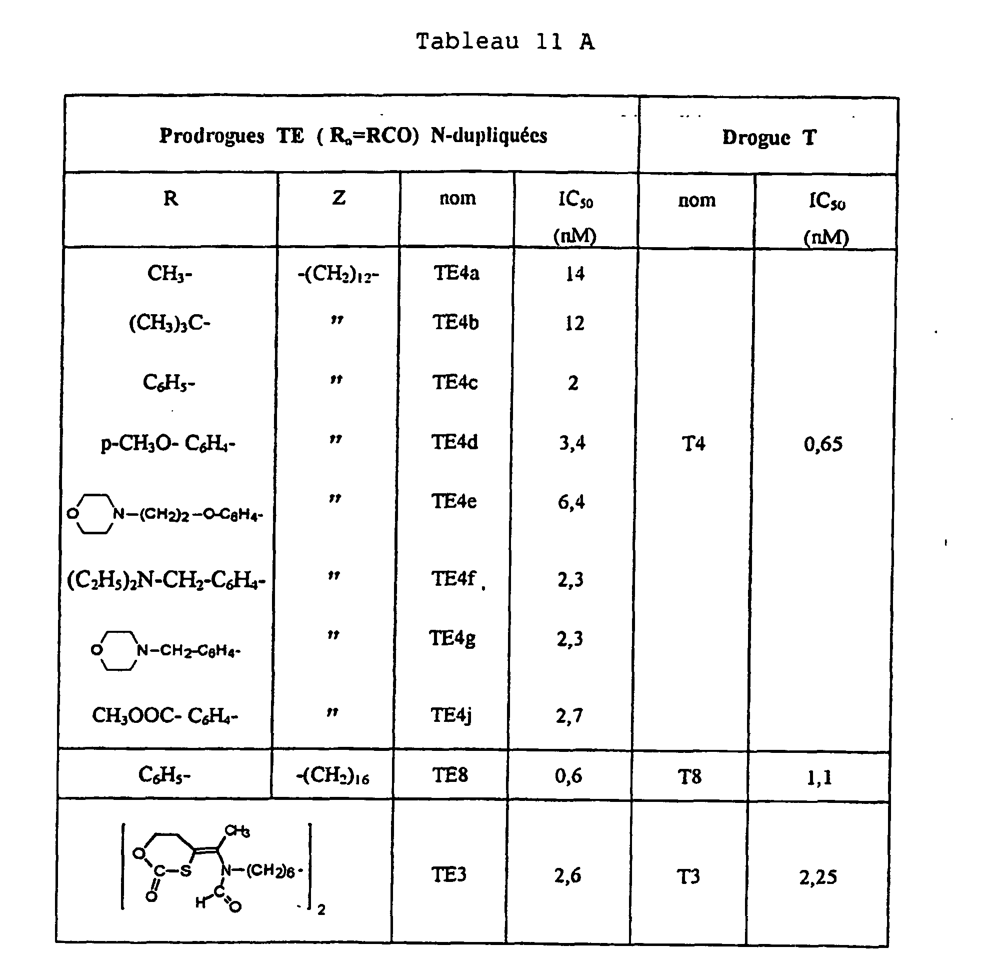

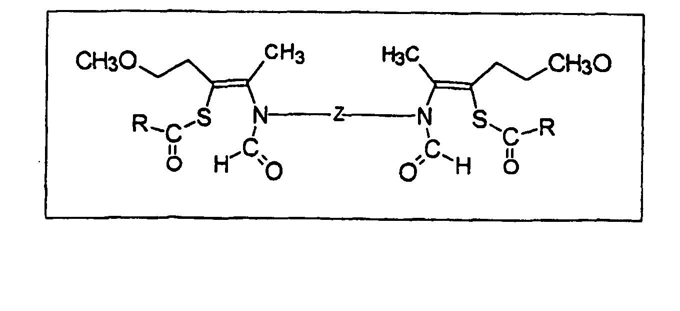

**Tableau 11B**

| **Prodrogues TE , 'R**_{**a**}**=RCO) C-dupliquées** | | | | | | **Drogue T** | |
|---|---|---|---|---|---|---|---|
| R | R₁ | R₂ | Z | nom | IC₅₀ (nM) | nom | IC₅₀ (nM) |
| p-CH₃O-C₆H₄- | C₆H₅-CH₂ | CH₃- | -(CH₂)₂O(CH₂)₆O(CH₂)₂- | TE10 | 12 | T10 | 2,5 |
| C₆H₃- | CH₃- | CH₂CO₂Et | -(CH₂)₁₂- | TE12 | 16 | T12 | 13 |

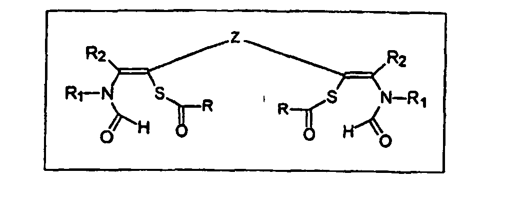

Ces résultats montrent que les valeurs de CI₅₀ obtenues sont très faibles aussi bien dans la série disulfure que dans la série thioester et sont de l'ordre de 1 à 14 nM pour les dérivés de type bis avec un bras espaceur formé par une chaîne dodécyle.

On remarquera avec intérêt que la valeur de CI₅₀ pour les composés cyclisés ionisés est sensiblement du même ordre de grandeur que celle des prodrogues neutres correspondantes.

A titre de comparaison, on a mesuré la valeur de CI₅₀ sur un composé ne pouvant pas se cycliser par hydrolyse enzymatique et répondant à la formule

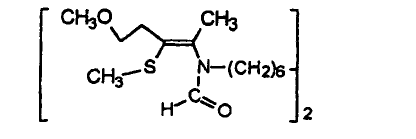

On obtient une valeur de CI₅₀ > 10⁻⁵ M ce qui indique que la cyclisation est nécessaire pour une forte activité antipaludique et suggère également que la cyclisation s'est effectivement produite en présence de sérum et/ou des érythrocytes infectés durant les 48 heures du test *in vitro* de mesure de l'activité antipaludique.

Dans le tableau 12 ci-après, on donne les résultats des mesures de CI₅₀ effectuées sur des haloalkylamines selon l'invention. Ces résultats concernent les prodrogues appelées P1 et P2 et les dérivés cyclisés correspondants G26 et G27 respectivement.

**Tableau 12**

| Nom | Z | R'₁ | R₁ | n | W | CI₅₀(nM) |
|---|---|---|---|---|---|---|
| P1 | -(CH₂)16- | H | CH₃ | 4 | Cl | 1,7 |
| G26 | " | " | " | " | I | 0,55 |
| P2 | " | CH₃ | " | 3 | Cl | 0,5 |
| G27 | " | " | " | " | I | 1,4 |

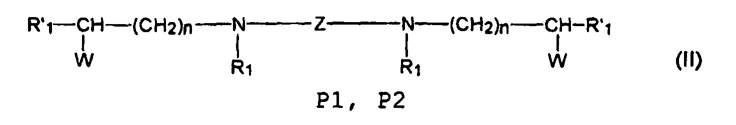

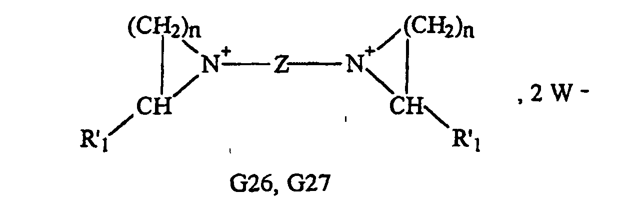

### B) Activité antipaludique in vivo chez la souris infectée par P. vinckei et tolérance après administration dans des conditions aigues ou semi-chroniques.

On rapporte dans le tableau 13 ci-après, les résultats obtenus avec des prodrogues selon l'invention de type disulfure (TS3b), de type thioester (TE4c, TE4a et TE4e), la drogue correspondante T3 et, à titre de comparaison, le dérivé d'ammonium quaternaire G25.

**Tableau 13**

| **Drogue** | ***In vitro*** **CI**_{**50**} **(nM) (*****P. falciparum*****)** | ***In vivo*** **DL**_{**50**} **(mg/kg) (souris)** | | **Indice d' absorption orale** ^{**c**} | ***In vivo*** **DE**_{**50**} **(mg/kg)** ***[TI](P. vinckei)*** |
|---|---|---|---|---|---|
| | | **aigûes**^{**a**} | **semichronique**^{**b**} | | |
| **G25** | **0,6** | **ip 1,27** | **1,15** | **87** | **0,22** ***[5,2]*** |
| | | **po 130** | **65** | | **~15** ***[4,4]*** |
| **T3** | **2,25** | **ip 10** | **7,5** | **70** | **0,2 [38]** |
| | | **po 700** | **160** | | |
| **TS3b** | **1,3** | **ip 240** | **90 (32*)** | **6,7** | **~7** ***[4,6*<TI≤12,8]*** |
| | | **po 1600** | **410** | | **~180** ***[2,3]*** |
| **TE4c** | **2** | **ip 100** | **≥ 30**^{**d**} | **~10** | **1** ***[≥ 30]***^{***a***} |
| | | **po ~1000** | **~300** | | **30 > x > 1** ***[10<TI<100]***^{***e***} |
| **TE4a** | **14** | **ip ~50** | **n.d.** | **~20** | |
| | | **po ~1000** | | | |
| **TE4e** | **6,4** | **ip ~50** | | | |

La CI₅₀ est la concentration qui inhibe de 50 % la croissance *in vitro* de *P. falciparum* : la DL₅₀ est la dose léthale correspondant à la mort de 50 % des souris et la DE₅₀ est la dose efficace pour inhiber de 50 % la croissance *in vivo* de *P. vinckei* selon un test suppressif de 4 jours, IT correspond à l'indice thérapeutique, IT = DL₅₀ (semi-chronique)/DE₅₀ ; ip: administration intra-péritonéale ; po : per os.
Dans ce tableau, a) à e) ont les significations suivantes:
a) : correspond à une seule dose ;
b) correspond à une administration deux fois par jour, pendant 4 jours consécutifs ;
c) correspond au rapport DL₅₀ po/DL50 ip dans des conditions d'administration aigüe, rapport appelé ci-après "indice d'absorption orale" ;
d) correspond à la mort de seulement 25 % des souris. "*" La DL₅₀ (semi-chronique) diminue chez les souris infectées par le paludisme ;
e) TE4c a été utilisé dans un mélange 50/50 de PEG/huile de castor.

Ces résultats montrent que les prodrogues de l'invention possèdent une forte activité antipaludique *in vitro* et *in vivo* ainsi qu'une bonne tolérance et une absorption élevées.

### C) Caractéristiques pharmacocinétiques et taux sérique.

### C1. Paramètres pharmacocinétiques chez la souris

On rapporte ci-après les résultats des paramètres pharmacocinétiques après administration par voie intra-péritonéale ou orale chez la souris pour une prodrogue de type disulfure (TS3b) et une prodrogue de type thioester (TE4c).

Pour la détermination du niveau sérique, on utilise des bio-essais *ex vivo* : brièvement, le médicament est administré à l'animal, puis on procède à des prélèvements sanguins répétés. Les *sera* sont décomplémentés pendant 30 min à 56°C. Le contenu en métabolite actif est alors déterminé par incubation de différentes concentrations (dilution dichotomique) de chaque sérum, en présence de suspensions d'érythrocytes infectés par *P. falciparum*, selon la méthode de DESJARDINS à la [³H] hypoxanthine.

Les résultats sont exprimés en SI₅₀, ce qui correspond au pourcentage de sérum (contenant un métabolite actif) capable d'inhiber la croissance de *P. falciparum* de 50 %.

Cette valeur est alors transformée en concentration sérique, (habituellement exprimée en ng/ml) en testant le composé actif directement (sans passage chez l'animal), sur la même suspension infectée par *P. falciparum* et en déterminant sa valeur de CI₅₀ (en ng/ml) [taux sérique = CI₅₀] (en ng/ml) x 100/SI₅₀ (en %)].

Les résultats sont exprimés en log (taux sérique de médicaments), en fonction du temps, ce qui permet l'évaluation du demi-temps pour la distribution vers le compartiment sérique t_{1/2(d)} ; du demi-temps pour l'élimination du compartiment sérique (t_{½(e)}) ; de C₀), correspondant au taux sérique extrapolé à l'origine dans la phase d'élimination ; de AUC (qui indique la quantité de drogue circulant dans le courant sanguin) ; et la biodisponibilité relative dans le mode d'administration par voie orale, versus le mode par voie intrapéritonéale [AUC (po)/AUC (ip)] qui est significatif du degré d'absorption par voie orale.

### . Pharmacocinétiques de TE4c

On administre à des souris des doses de 3 et 50 mg par kg de TE4c, par voie intrapéritonéale et par voie orale, ce qui correspond à des DL₅₀/33 et DL₅₀/20 respectivement.

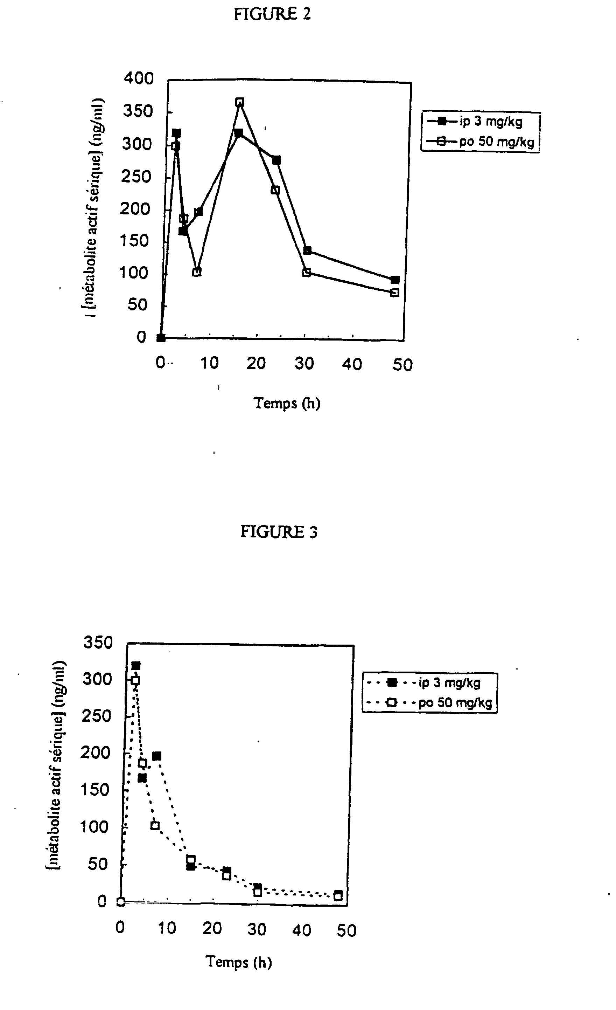

Le composé est solubilisé dans du DMSO à 10 %. Même à ces faibles doses, on observe des taux sériques élevés avec un profil pharmacocinétique bi-phasique pour les deux voies. Les résultats sont donnés sur la figure 2 qui indique la concentration en métabolite actif sérique en ng/ml) en fonction du temps en heures.

Sur cette figure, (-v-) correspond à l'administration ip à 3 mg/kg et la courbe avec (-o-) à l'administration po à 50 mg/kg.

Dans la première phase, on observe très rapidement un pic (en moins de deux heures) avec des taux sériques diminuant jusqu'à 4 à 7 heures, puis augmentant et on note à nouveau un pic autour de 15 heures, et ceci pour les deux voies d'administration.

Ces résultats suggèrent une première phase rapide au cours de laquelle la prodrogue est distribuée et entre dans le compartiment sérique. Une fois dans ce compartiment, la prodrogue se transforme vraisemblablement en composé ammonium quaternaire.

La deuxième phase peut alors être exploitée pour déterminer les paramètres pharmacocinétiques du dérivé ionisé.

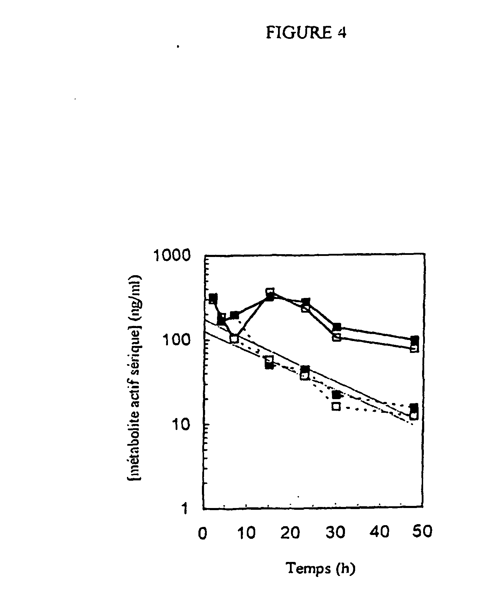

En considérant que la transformation en drogue ionisée est totale, le profil pharmacocinétique peut être redessiné sur la base de la CI₅₀ de la drogue et non sur celui de la prodrogue (voir figure 3, où les légendes des courbes sont les mêmes que dans la figure 2). La représentation semi-logarithmique permet de déterminer les paramètres pharmacocinétiques principaux du métabolite actif (qui est supposé être la drogue ammonium quaternaire T4) pour les deux voies d'administration (voir figure 4 où les résultats sont redessinés à partir des figures 2 et 3). Les paramètres pharmacocinétiques sont C₀ = 180 ng/ml, t_{1/2} = 12 heures, AUC = 3,3 µg.h/ml après administation ip à 3 mg/kg, et Cₒ = 130 ng/ml, t_{½} = 12 h 30, AUC = 2,7 µg.h/ml après administration orale à 50 mg/kg. Dans ces conditions, la biodisponibilité relative est de 5 %.

Dans une autre série d'expériences, on a étudié la pharmacocinétique à des doses plus élevées de TE4c (10 mg/kg ip et 150 mg/kg po).

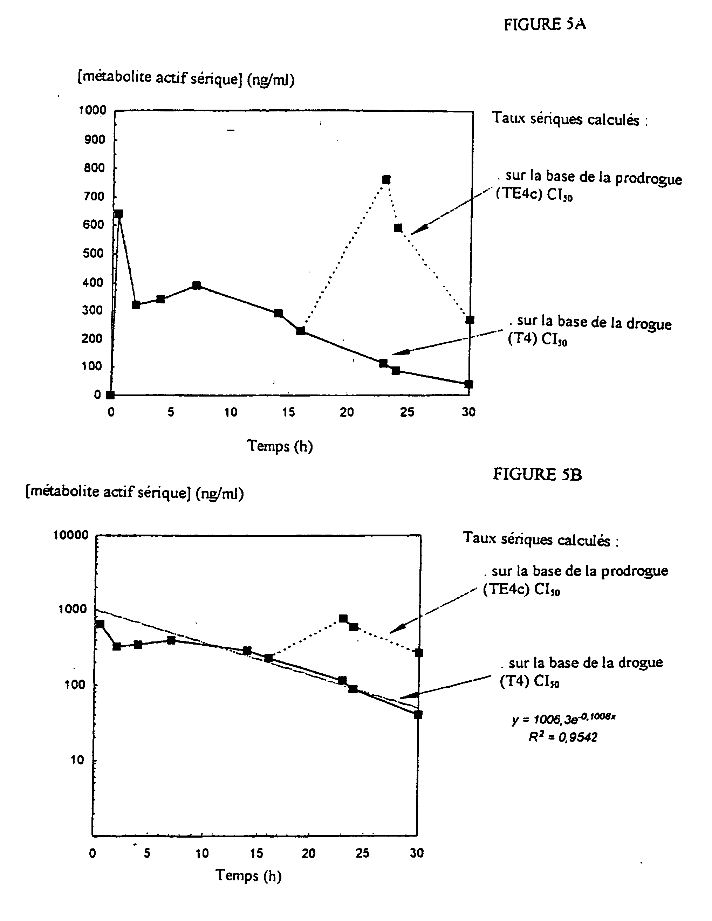

Un profil biphasique a également été observé avec des taux sériques plus élevés et dans ce cas le second pic s'est produit légèrement plus tard (autour de 23 h) comme le montrent les figures 5A et 5B.

### Estimation des paramètres pharmacocinétiques : par voie orale,

C₀ = 1 µg/ml et t_{1/2} autour de 10 heures.

### . Pharmacocinétique de TS3b

TS3b a été administré à la souris à des doses de 50 et 400 mg/kg, respectivement par voie intrapéritonéale et par voie orale (DL₅₀/3 approximativement).

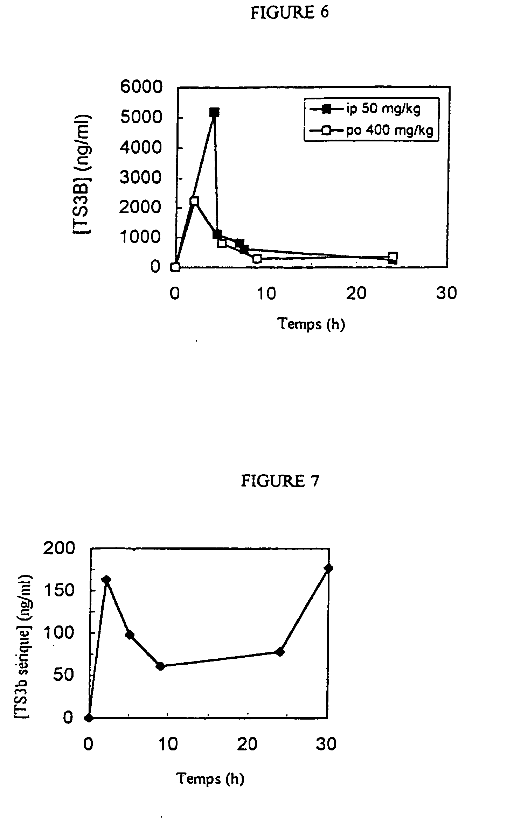

On observe des taux sériques élevés formant des pics 2 à 4 heures après l'administration de drogues (figure 6). Les paramètres pharmacocinétiques ont été alors estimés à partir de la représentation semi-logarithmique de la concentration sérique (calculée sur la base de la CI₅₀ de TS3b en fonction du temps).

Après administration intra-péritonéale à 50 mg/kg, on obtient C₀ de 2,75 µg/ml avec t_{1/2} d'environ 6 heures.

Par voie orale à 400 mg/kg, C₀ est de 1,8 µg/ml, indiquant de très hauts taux sériques. Le t_{1/2} apparent, est de 13 heures. Une telle différence dans les t_{1/2} entre les deux voies d'administration pourrait indiquer une différence de métabolisation de la prodrogue.

Le profil de pharmacocinétique observé sur la figure 6, pourrait refléter seulement la première phase d'un profil biphasique, correspondant à une phase absorption /distribution, comme celle observée pour la prodrogue de type thioester TE4c.

Une autre série d'expériences a été réalisée pour vérifier l'existence d'un profil biphasique, avec des prélèvements sanguins jusqu'à 30 heures après l'administration de la drogue par voie ip (voir figure 7 qui donne la concentration de TS3b sérique en ng/ml en fonction du temps en heures).

On constate qu'on obtient bien un profil biphasique, bien qu'incomplet.

La première phase se caractérise par un pic à environ 2 heures avec des taux sériques diminuant jusqu'à 10 heures; entre 10 et 24 heures, on n'observe qu'une augmentation légère suivie d'une remontée brutale entre 24 h et 30 heures. Ceci correspond vraisemblablement à une première phase dans laquelle la prodrogue est distribuée et entre dans le compartiment sérique.

Cette première phase apparaît moins tôt que pour TE4C considéré ci-dessus.

Une fois dans le compartiment sérique, la transformation de la prodrogue en composé quaternaire se produit. Des expériences pharmacocinétiques à très long terme doivent être réalisées pour évaluer les paramètres de pharmacocinétique de la deuxième phase (c'est-à-dire du dérivé ionisé correspondant à T3) ce qui a conduit à utiliser comme modèle le singe.

### Paramètres pharmacocinétiques chez le singe.

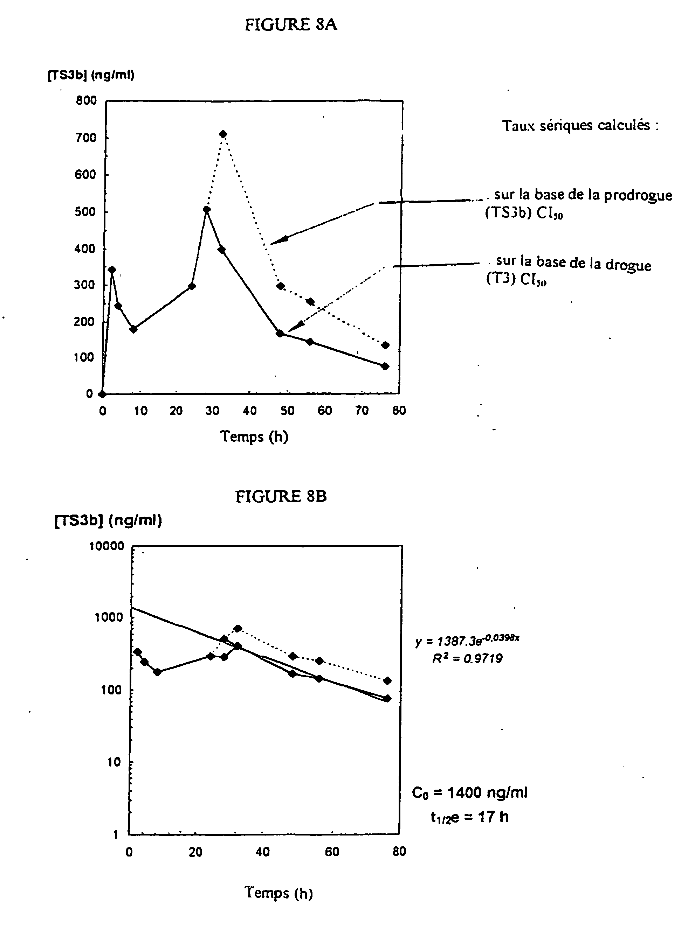

On administre par voie intramusculaire TS3b à des singes *Macaca fascicularis* à 4 mg/kg (voir figures 8A et 8B qui donnent la concentration en TS3b en ng/ml en fonction du temps en heures).

On effectue des prélèvements sanguins répétés jusqu'à 76 heures et on observe un profil biphasique net.

La première phase forme un pic très rapidement en moins de deux heures avec des taux sériques diminuant jusqu'à 10 heures, puis augmentant et formant un pic à nouveau autour de 30 heures.

Comme indiqué ci-dessus, ceci peut correspondre à la première phase rapide pendant laquelle la prodrogue est distribuée et entre dans le compartiment sérique.

Une fois dans ce compartiment, la prodrogue se transforme en composés ammonium quaternaires.

La seconde phase peut alors être exploitée pour déterminer les paramètres de pharmacocinétique du dérivé ionisé correspondant à T3.

En considérant que la transformation complète en drogue ionisée s'est produite, le profil pharmacocinétique peut être redessiné ci-dessus sur la base de la CI₅₀ de la drogue et non sur celui de la prodrogue (voir figure 8A). On observe des taux sériques élevés, C₀ est de 1,4 µg/ml et t_{1/2} autour de 17 heures.

L'ensemble de ces résultats met en évidence des propriétés pharmacocinétiques des différents produits appropriées pour en exploiter les différentes activités pharmacologiques revendiquées.

### Activité antipaludique contre Plasmodium falciparum chez le singe Aotus.

3 singes *Aotus* ont été infectés par *P. falciparum* (souche FVO). Lorsque la parasitémie sanguine a atteint 1 % (2 singes) ou 6 % (1 singe), un traitement par voie intramusculaire à raison de 2 injections par jour de TE4c (2mg/kg), pendant 8 jours, a été pratiqué. Dans chacun des cas, la parasitémie sanguine a été complètement éliminée et aucune recrudescence n'a été observée dans les 6 mois suivant le traitement. Ces résultats indiquent une capacité bien réelle du composé à guérir le paludisme humain du à *P. falciparum*.

### Activités antibabésioses des composés

Les produits TE4c, TS3b et P1 ont aussi été évalués *in vitro* pour leurs activités contre Babésia divergens et *B. canis*. Dans l'un et l'autre cas, les composés TE4c, TS3b et P1 se sont montrés particulièrement actifs (CI₅₀ < 20 nM). Ces résultats indiquent une activité antibabésia puissante pour ce type de composés.

## Revendications

1. Précurseurs de drogues à effet anti-paludique, **caractérisés en ce qu'**il s'agit de produits capables de générer sels de bis-ammonium quaternaire et qu'ils répondent à la formule générale (I) 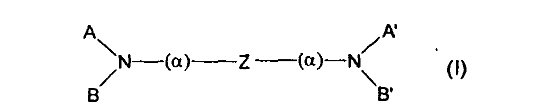 dans laquelle
- A et A' sont identiques ou différents l'un de l'autre et représentent
. soit, respectivement, un groupe A₁ et A'₁ de formule 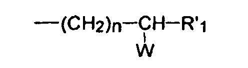 où n est un entier de 2 à 4 ; R'₁ représente un atome d'hydrogène, un radical alkyle en C1 à C5, éventuellement substitué par un radical aryle (notamment un radical phényle), un hydroxy, un alkoxy, dans lequel le radical alkyle comprend de 1 à 5 C, ou aryloxy (notamment phénoxy) ; et W représente un atome d'halogène choisi parmi le chlore, le brome ou l'iode, ou un groupe nucléofuge, comme le radical tosyle CH₃-C₆H₄-SO₃, mésityle CH₃-SO₃, CF₃-SO₃, NO₂-C₆H₄-SO₃,
. soit un groupe A₂ qui représente un radical formyle -CHO, ou acétyle -CO-CH₃,
- B et B' sont identiques ou différents l'un de l'autre et représentent
. soit respectivement les groupes B₁ et B'₁, si A et A' représentent respectivement A₁ et A'₁, B₁ et B'₁ représentant un groupe R₁ qui présente la même définition que R'₁ ci-dessus, mais ne peut pas être un atome d'hydrogène,
. soit respectivement les groupes B₂ et B'₂, si A et A' représentent A₂, B₂ ou B'₂ étant le groupe R₁ tel que défini ci-dessus, ou un groupement de formule 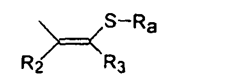 dans lequel -Ra représente un groupe RS- ou RCO-, où R est un radical alkyle en C1 à C6, notamment de C1 à C5, linéaire, ramifié, ou cyclique, le cas échéant substitué par un ou plusieurs groupes hydroxy, alcoxy ou aryloxy, ou un groupe amino et/ou un groupe -COOH ou COOM, où M est un alkyle en C1 à C3 ; un radical phényle ou benzyle, dans lequel le radical phényle est le cas échéant substitué par au moins un radical alkyle ou alcoxy en C1 à C5, ceux-ci étant éventuellement substitués par un groupe amino, ou par un hétérocycle azoté ou oxygéné, un groupe -COOH ou -COOM; ou un groupe -CH₂-hétérocycle, à 5 ou 6 éléments, azoté et/ou oxygéné ; R₂ représente un atome d'hydrogène, un radical alkyle en C1 à C5, ou un groupe -CH₂-COO-alkyl(C1 à C5); et R₃ représente un atome d'hydrogène, un radical alkyle ou alkényle en C1 à C5, le cas échéant substitué par -OH, un groupement phosphate, un radical alkoxy, dans lequel le radical alkyle est en C1 à C3, ou aryloxy; ou un groupe alkyl (ou aryl) carbonyloxy; ou encore R₂ et R₃ forment ensemble un cycle à 5 ou 6 atomes de carbone ; R et R₃ peuvent être reliés pour former un cycle à 5 à 7 atomes (carbone, oxygène, soufre)
- α représente
. soit une simple liaison, lorsque A et A' représentent A₁ et A'₁ : ou lorsque A et A' représentent A₂ , c'est-à-dire un groupe -CHO ou -COCH₃, et B₂ et B'₂ représentent 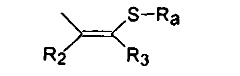
. soit, lorsque A et A' représentent A₂ et B₂ et B'₂ représentent R₁ , un groupement de formule, 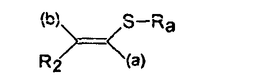 ou un groupement de formule 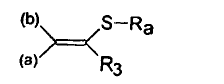 dans lesquels (a) représente une liaison vers Z et (b) une liaison vers l'atome d'azote,
- Z représente un radical alkyle en C6 à C21, notamment de C13 à C21 le cas échéant avec insertion d'une ou de plusieurs liaisons multiples, et/ou d'un ou plusieurs hétéroatomes O et/ou S, et/ou d'un ou de plusieurs cycles aromatiques, et les sels pharmaceutiquement acceptables de ces composés, sous réserve que R'₁ ne représente pas H ou un radical alkyle en C1 ou C2, lorsque n = 3 ou 4, R₁ représente un radical alkyle en C1 à C4 et Z représente un radical alkyle en C6 à C10.

2. Précurseurs selon la revendication 1, **caractérisés en ce qu'**il s'agit d'haloalkylamines, répondant à la formule générale (II) 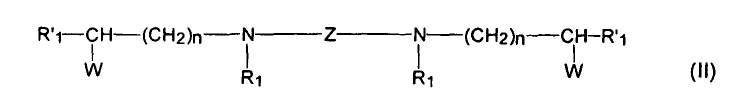 dans laquelle R₁, R'₁, W, n et Z sont tels que définis dans la revendication 1.

3. Précurseurs selon la revendication 1,
**caractérisé en ce que** Z représente un radical alkyle de C13 à C21.

4. Précurseurs selon la revendication 3, **caractérisés en ce que** Z représente un groupe -(CH₂)₁₆ -.

5. Précurseurs selon l'une quelconque des revendications revendication 2 à 4, **caractérisés en ce que** R₁ est un radical méthyle.

6. Précurseurs selon l'une quelconque des revendications 2 à 5, **caractérisés en ce que** R₁ est un radical méthyle et R'₁ est soit un atome d'hydrogène, soit un radical méthyle, et W est un atome de chlore.

7. Précurseurs selon l'une quelconque des revendications 2 à 6, **caractérisés en ce qu'**ils sont choisis parmi le dichlorhydrate du N, N'-diméthyl-N,N'-(5-chloropentyl)-1,16-hexadécanediamine, ou le dichlorhydrate du N, N'-diméthyl-N,N'-(4-chloropentyl)-1,16-hexadécanediamine.

8. Précurseurs selon la revendication 1, **caractérisés en ce qu'**il s'agit de précurseurs de thiazolium répondant à la formule générale (III). 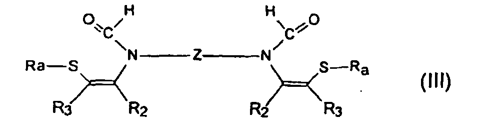 ou à la formule générale (IV) 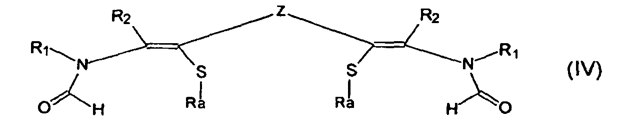 ou à la formule générale (V) 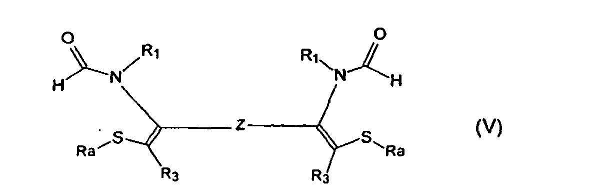 dans laquelle Rₐ, R₁, R₂, et Z sont tels que définis dans la revendication 1.

9. Précurseurs selon la revendication 8, **caractérisés en ce qu'**ils répondent à la formule III dans laquelle Rₐ représente un radical RCO-.

10. Précurseurs selon la revendication 9, **caractérisés en ce qu'**il sont choisis parmi le N,N'-diformyl-N,N'-di[1-méthyl-2-S-thiobenzoyl-4-méthoxybut-1-ényl]-1, 12-diaminododécane, le N,N'-diformyl-N,N'-di [1-méthyl-2-S-(p-diéthylaminométhylphényl-carboxy)thio-4-méthoxybut-1-ényl]-1, 12-diaminododécane, le N,N'-diformyl-N,N'-di[1-méthyl-2-S-(p-morpholino-méthylphényl carboxy)-thio-4-méthoxybut-1-ényl]-1,12-diaminododécane, et le N,N'-diformyl-N,N'-di [1-méthyl-2-S-thiobenzoyl-4-méthoxybut-1-ényl]-1, 16- diamino hexadécane, et le N, N'-diformyl-N, N'-di[1(2-oxo-4,5-dihydro-1,3-oxathian-4-ylidène) éthyl]1,12-diaminododécane.

11. Précurseurs selon la revendication 8, **caractérisés en ce que** Rₐ représente RS-.

12. Précurseurs selon la revendication 11, **caractérisés en ce qu'**ils sont choisis parmi
N,N'-diformyl-N,N'-di[1-méthyl-2-tétrahydrofurfuryl-méthyldi thio-4-hydroxybut-1-ényl]-1,12-diaminododécane,
N,N'-diformyl-N,N'-di[1-méthyl-2-propyl-dithio-4- hydroxybut-1-ényl]-1, 12- diaminododécane,
N,N'-diformyl-N,N' -di[1-méthyl-2-benzyl-dithio- 4-hydroxybut-1- ényl]-1, 12 diaminododécane,
N,N'-diformyl-N,N' -di[1-méthyl-2-(2-hydroxyéthyldithio- 4-hydroxybut-1- ényl]-1, 12-diaminododécane,
N,N'-diformyl-N,N'-di[1-méthyl-2-propyldithio-4-méthoxybut-1-ényl]-1, 12-diaminododécane, et
N,N'-diformyl-N,N'-di[1-méthyl-2-propyldithio-éthényl] -1,12-diaminododécane.

13. Précurseurs selon la revendication 8, **caractérisés en ce qu'**ils répondent à la formule IV et sont choisis parmi le 2,17-(N,N'-diformyl- N,N'-diméthyl)diamino-3,16-S-thio-p-méthoxybenzoyl-6,13-dioxaoctadéca- 2,16-diène, le 2,17-(N,N'-diformyl- N,N'-dibenzyl)diamino-3,16-S-thio-p-méthoxybenzoyl- 6,13-dioxaoctadéca- 2,16- diène, le 3,18 (N,N'- diformyl- N,N'-diméthyldiamino-4,17-S-thiobenzoyl-eicosa-3,17-diènedioate d'éthyle (TE12), le 3,18-(N,N'-diformyl-N,N'-dibenzyl)diamino-4,17-S-thiobenzoyl-eicosa-3,17-diènedioate d'éthyle.

14. Précurseurs selon la revendication 8, **caractérisés en ce qu'**ils répondent à la formule (V) et sont choisis parmi le 2,15-(N,N'-diformyl-N,N'-diméthyl)diamino-1,16-S-thiabenzoyl-hexadéca-1,15-diène, le 2,15-(N,N'-diformyl-N,N'-dibenzyl)diamino-1,16-S-thio-benzoyl-hexadéca-1,15-diène.

15. Les dérivés cyclisés correspondant aux précurseurs de thiazolium selon l'une quelconque des revendications 8 à 14, répondant à la formule générale (VI) 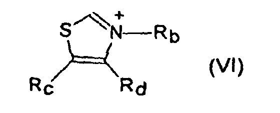 dans laquelle
. R_{b} représente R₁ ou T, T représentant le groupe de formule : 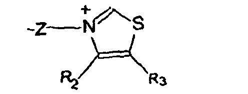 sous réserve que Z ne représente pas un radical alkyle en C6 à C8, lorsque R_{c}, R_{d}, R₂ et R₃ représentent un radical méthyle, ou lorsque R_{c} et R_{d} d'une part, et R₂ et R₃ d'autre part, forment ensemble des cycles aromatiques à 6 atomes de carbone,
. R_{d} représente R₂ ou P, P représentant le groupe de formule 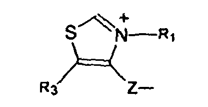
. R_{c} représente R₃ ou U, U représentant le groupe de formule 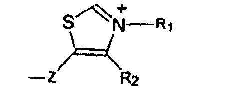
R₁, R₂, R₃ et Z étant tels que définis dans la revendication 1,
étant entendu que R_{b} = T, si R_{c} = R₃ et R_{d} = R₂ ; R_{d} = P, si R_{c} = R₃ et R_{b} = R₁ ; et R_{c} = U, si R_{b} = R₁, et R_{d} = R₂.

16. Procédé d'obtention de précurseurs de thiazolium de formule générale (III) ou (IV) selon la revendication 8, **caractérisé en ce qu'**il comprend la réaction en milieu basique d'un dérivé de thiazole de formule (VI).

17. Procédé selon la revendication 16, **caractérisé en ce que** pour obtenir les composés dans lesquels Rₐ = RCO-, on fait réagir un dérivé de thiazolium de formule (VI) avec un dérivé RCOR', où R est tel que défini dans la revendication 1 et R' est un atome d'halogène, et pour obtenir les composés dans lesquels Rₐ = RS-, on fait réagir lesdits dérivés de thiazolium de formule (VI) avec un dérivé de thiosulfate RS₂-O₃Na.

18. Procédé selon la revendication 16 ou 17, **caractérisé en ce que**
- pour obtenir les composés de formule (III) on fait réagir un dérivé de thiazole convenablement substitué avec un dihalogénure d'alkyle, à reflux dans un solvant organique, l'ouverture du cycle thiazolium se faisant ensuite en milieu basique, et par action soit de R-COCl, soit de R-S₂O₃Nₐ,
- pour obtenir les composés de formule IV, qui comportent un oxygène dans la chaîne Z, on fait réagir un dérivé de thiazole de formule générale (VII) 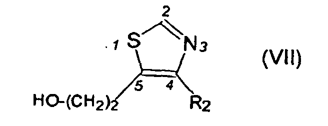 avec un dihalogénure d'alcane, en milieu basique, puis l'addition de R₁X, le milieu réactionnel étant avantageusement porté à reflux dans un solvant organique, notamment alcoolique comme l'éthanol, pendant une durée suffisante pour obtenir la quaternisation de l'atome d'azote du thiazole par fixation de R₁, l'ouverture du cycle thiazolium étant obtenue ensuite en milieu basique, puis par action soit de R-COCl, soit de R-S₂O₃Na,
- pour obtenir les composés de formule (IV) ne comportant pas d'oxygène dans la chaîne Z, on synthétise tout d'abord un composé de structure 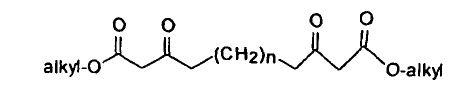 par réaction d'un acétoacétate d'alkyle avec NaH, suivie d'une alkylation, puis de l'addition d'un dihalogénoalcane, le composé obtenu étant ensuite dibromé, puis additionné de thioformamide et, après reflux plusieurs jours, de R₁X, ce qui conduit, après un nouveau reflux pendant plusieurs jours, à un thiazolium dont l'ouverture est ensuite réalisée en milieu basique, puis action de R-COCl ou de R-S₂O₃Na,
- pour obtenir les composés de formule (V) ne comportant pas d'oxygène dans la chaîne Z, on fait réagir un composé Z(CO-CH₂ X)₂ avec CH(=S)NH₂, puis on ajoute R₁X, l'ouverture du cycle thiazolium étant ensuite réalisée en milieu basique, puis en ajoutant R-COCl ou R-S₂O₃Nₐ.

19. Procédé d'obtention d'haloalkylamines selon la revendication 1, **caractérisé en ce qu'**il comprend l'alkylation d'un aminoalcool de formule : 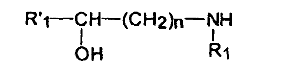 par un α, ω-dihalogénure d'alkyle X-Z-X, ce qui conduit à un bis-aminoalcool traité par un composé capable de libérer le groupe W.

20. Compositions pharmaceutiques, **caractérisées en ce qu'**elles renferment une quantité efficace d'au moins un précurseur tel que défini dans l'une quelconque des revendications 1 à 14, ou au moins un dérivé cyclisé correspondant aux précurseurs de thiazolium de formule générale (VI) : 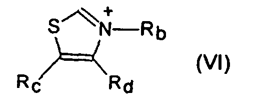 dans laquelle
. R_{b} représente R₁ ou T, T représentant le groupe de formule : 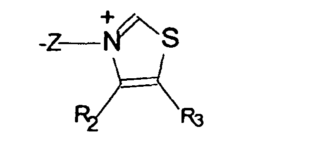
. R_{d} représente R₂ ou P, P représentant le groupe de formule 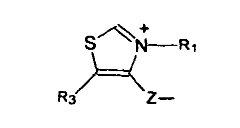
. R_{c} représente R₃ ou U, U représentant le groupe de formule 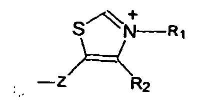
R₁, R₂, R₃ et Z étant tels que définis dans la revendication 1,
étant entendu que R_{b} = T, si R_{c} = R₃ et R_{d} = R₂ ; R_{d} = P, si R_{c} = R₃ et R_{b} = R₁ ; et R_{c} = U, si R_{b} = R₁, et R_{d} = R₂,
en association avec un véhicule pharmaceutiquement inerte.

21. Utilisation pour la fabrication de médicaments pour le traitement des maladies infectieuses, en particulier du paludisme ou des babésioses chez l'homme ou l'animal, de sels de bis-ammonium quaternaire de formule générale I 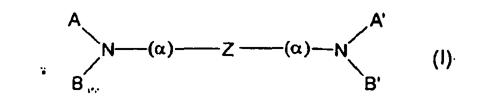 dans laquelle
- A et A' sont identiques ou différents l'un de l'autre et représentent
. soit, respectivement, un groupe A₁ et A'₁ de formule 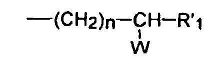 où n est un entier de 2 à 4 ; R'₁ représente un atome d'hydrogène, un radical alkyle en C1 à C5, éventuellement substitué par un radical aryle (notamment un radical phényle), un hydroxy, un alkoxy, dans lequel le radical alkyle comprend de 1 à 5 C, ou aryloxy (notamment phénoxy) ; et W représente un atome d'halogène choisi parmi le chlore, le brome ou l'iode, ou un groupe nucléofuge, comme le radical tosyle CH₃-C₆H₄-SO₃, mésityle CH₃-SO₃, CF₃-SO₃, NO₂-C₆H₄-SO₃,
. soit un groupe A₂ qui représente un radical formyle -CHO, ou acétyle- COCH₃,
- B et B' sont identiques ou différents l'un de l'autre et représentent
. soit respectivement les groupes B₁ et B'₁, si A et A' représentent respectivement A₁ et A'₁ , B₁ et B'₁ représentant un groupe R₁ qui présente la même définition que R'₁ ci-dessus, mais ne peut pas être un atome d'hydrogène,
. soit respectivement les groupes B₂ et B'₂, si A et A' représentent A₂, B₂ ou B'₂ étant le groupe R₁ tel que défini ci-dessus, ou un groupement de formule 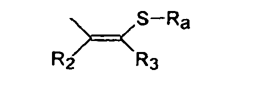 dans lequel -Ra représente un groupe RS- ou RCO-, où R est un radical alkyle en C1 à C6, linéaire, ramifié ou cyclique, le cas échéant substitué par un ou plusieurs groupes hydroxy, alcoxy ou aryloxy, ou un groupe amino et/ou un groupe -COOH ou COOM, où M est un alkyle en C1 à C3 ; un radical phényle ou benzyle, dans lequel le radical phényle est le cas échéant substitué par au moins un radical alkyle ou alcoxy en C1 à C5, ceux-ci étant éventuellement substitués par un groupe amino, ou par un hétérocycle azoté ou oxygéné, un groupe -COOH ou -COOM; ou un groupe -CH₂-hétérocycle, à 5 ou 6 éléments, azoté et/ou oxygéné ; R₂ représente un atome d'hydrogène, un radical alkyle en C1 à C5, ou un groupe -CH₂-COO-alkyl(C1 à C5); et R₃ représente un atome d'hydrogène, un radical alkyle ou alkényle en C1 à C5, le cas échéant substitué par -OH, un groupement phosphate, un radical alkoxy, dans lequel le radical alkyle est en C1 à C3, ou aryloxy; ou un groupe alkyl (ou aryl) carbonyloxy; ou encore R₂ et R₃ forment ensemble un cycle à 5 ou 6 atomes de carbone ; R et R₃ peuvent être reliés pour former un cycle de 5 à 7 atomes (carbone, oxygène, soufre)
- α représente
. soit une simple liaison, lorsque A et A' représentent A₁ et A'₁ : ou lorsque A et A' représentent A₂ , c'est-à-dire un groupe -CHO ou -COCH₃, et B₂ et B'₂ représentent 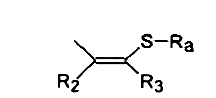
. soit, lorsque A et A' représentent un groupe -CHO et B₂ et B'₂ représentent R₁ , un groupement de formule : 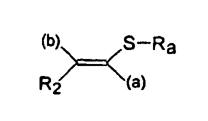 ou un groupement de formule : 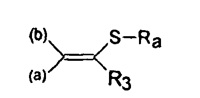 dans lesquels (a) représente une liaison vers Z et (b) une liaison vers l'atome d'azote,
- Z représente un radical alkyle en C6 à C21, le cas échéant avec insertion d'une ou de plusieurs liaisons multiples, et/ou d'un ou plusieurs hétéroatomes O et/ou S, et/ou d'un ou de plusieurs cycles aromatiques, et les sels pharmaceutiquement acceptables de ces composés.

22. Compositions pharmaceutiques selon la revendication 20, ou médicaments fabriqués selon la revendication 21, **caractérisés en ce qu'**ils sont administrables par voie orale, par voie injectable, ou encore par voie rectale.

## Claims

1. Precursors of drugs with an anti-malarial action, **characterized in that** these are products capable of generating quaternary bis-ammonium salts and that they correspond to general formula (I) 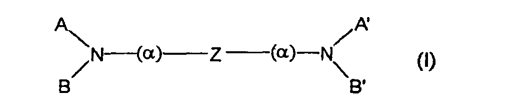 in which
- A and A' are identical to or different from one another and represent
. either, an A₁ and A'₁ group respectively, of formula 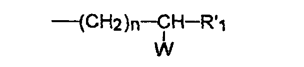 where n is an integer from 2 to 4; R'₁ represents a hydrogen atom, a C1 to C5 alkyl radical, optionally substituted by an aryl radical (in particular a phenyl radical), a hydroxy, an alkoxy, in which the alkyl radical comprises from 1 to 5 C, or aryloxy (in particular phenoxy); and W represents a halogen atom chosen from chlorine, bromine or iodine, or a nucleofuge group, such as the tosyl CH₃-C₆H₄-SO₃, mesityl CH₃-SO₃, CF₃'-SO₃, NO₂-C₆H₄-SO₃ radical,
. or an A₂ group which represents a formyl -CHO, or acetyl -COCH₃ radical,
- B and B' are identical to or different from one another and represent
. either the B₁ and B'₁ groups respectively, if A and A' represent A₁ and A'₁ respectively, B₁ and B'₁ representing an R₁ group which has the same definition as R'₁ above, but cannot be a hydrogen atom,
. or the B₂ and B'₂ groups respectively, if A and A' represent A₂, B₂ or B'₂ being the R₁ group as defined above, or a group of formula 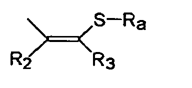 in which -Ra represents an RS- or RCO- group, where R is a linear, branched or cyclic C1 to C6, in particular C1 to C5, alkyl radical, optionally substituted by one or more hydroxy, alkoxy or aryloxy groups or an amino group and/or a -COOH or COOM group, where M is a C1 to C3 alkyl; a phenyl or benzyl radical, in which the phenyl radical is optionally substituted by at least one C1 to C5 alkyl or alkoxy radical, these being optionally substituted by an amino group, or by a nitrogenous or oxygenous heterocycle, a -COOH or -COOM group; or a -CH₂-heterocycle group, with 5 or 6 elements, nitrogenous and/or oxygenous; R₂ represents a hydrogen atom, a C1 to C5 alkyl radical, or a -CH₂-COO-alkyl (C1 to C5) group; and R₃ represents a hydrogen atom, a C1 to C5 alkyl or alkenyl radical, optionally substituted by -OH, a phosphate group, an alkoxy radical, in which the alkyl radical is C1 to C3, or an aryloxy radical; or an alkyl (or aryl), carbonyloxy group; or also R₂ and R₃ together form a ring with 5 or 6 carbon atoms; R and R₃ can be linked to form a ring of 5 to 7 atoms (carbon, oxygen, sulphur)
- α represents
. either a single bond, when A and A' represent A₁ and A'₁: or when A and A' represent A₂ , i.e. a -CHO or - COCH₃ group, and B₂ and B'₂ represent 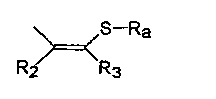
. or, when A and A' represent A₂ and B₂ and B'₂ represent R₁ , a group of formula 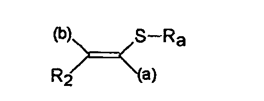 or a group of formula 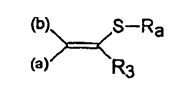 in which (a) represents a bond towards Z and (b) a bond towards the nitrogen atom.
- Z represents a C6 to C21 alkyl radical, in particular C13 to C21 optionally with insertion of one or more multiple bonds, and/or one or more O and/or S heteroatoms, and/or one or more aromatic rings, and the pharmaceutically acceptable salts of these compounds, on the condition that R'₁ does not represent H or a C1 or C2 alkyl radical, when n = 3 or 4, R₁ represents a C1 to C4 alkyl radical and Z represents a C6 to C10 alkyl radical.

2. Precursors according to claim 1, **characterized in that** these are haloalkylamines, corresponding to general formula (II) 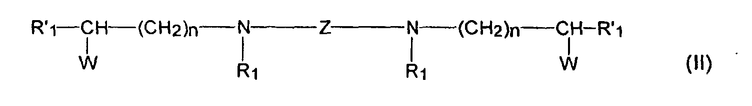 in which R₁, R'₁, W, n and Z are as defined in claim 1.

3. Precursors according to claim 1 or 2, **characterized in that** Z represents a C13 to C21 alkyl radical.

4. Precursors according to claim 3, **characterized in that** Z represents a -(CH₂)₁₆ - group.

5. Precursors according to any one of claims 2 to 4, **characterized in that** R₁ is a methyl radical.

6. Precursors according to any one of claims 2 to 5, **characterized in that** R₁ is a methyl radical and R'₁ is either a hydrogen atom, or a methyl radical, and W is a chlorine atom.

7. Precursors according to any one of claims 2 to 6, **characterized in that** they are chosen from N, N'-dimethyl-N,N'-(5-chloropentyl)-1,16-hexadecanediamine dihydrochloride, or N, N'-dimethyl-N,N'-(4-chloropentyl)-1,16-hexadecanediamine dihydrochloride.

8. Precursors according to claim 1, **characterized in that** these are precursors of thiazolium corresponding to general formula (III). 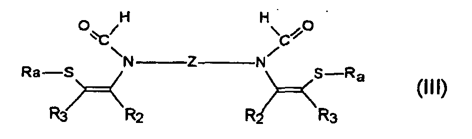 or to general formula (IV) 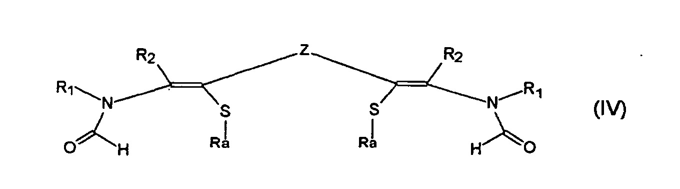 or to general formula (V) 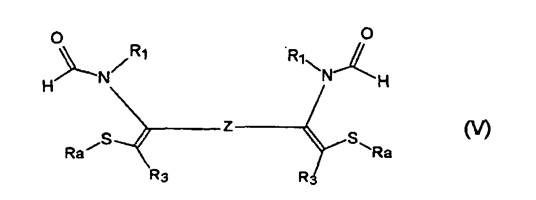 in which Rₐ, R₁, R₂, and Z are as defined in claim 1.

9. Precursors according to claim 8, **characterized in that** they correspond to formula III in which Rₐ represents an RCO- radical.

10. Precursors according to claim 9, **characterized in that** they are chosen from N,N'-diformyl-N,N'-di[1-methyl-2-5-thiobenzoyl-4-methoxybut-1-enyl]-1, 12-diaminododecane, N,N'-diformyl-N,N'-di[1-methyl-2-S-(p-diethylaminomethylphenylcarboxy)thio-4-methoxybut-1-enyl]-1,12-diaminododecane, N,N'-diformyl-N,N'-di[1-methyl-2-S-(p-morpholino-methylphenylcarboxy)-thio-4-methoxybut-1-enyl]-1,12-diaminododecane, and N,N'-diformyl-N,N'-di[1-methyl-2-S-thiobenzoyl-4-methoxybut-1-enyl]-1,16-diaminohexadecane and N,N'-diformyl-N,N'-di[1(2-oxo-4,5-dihydro-1,3-oxathian-4-ylidene)ethyl]1,12-diaminododecane.

11. Precursors according to claim 8, **characterized in that** Rₐ represents RS-.

12. Precursors according to claim 11, .**characterized in that** they are chosen from
N,N'-diformyl-N,N'-di[1-methyl-2-tetrahydrofurfuryl-methyldithio-4-hydroxybut-1-enyl]-1,12-diaminododecane,
N,N'-diformyl-N,N'-di[1-methyl-2-propyl-dithio-4-hydroxybut-1-enyl]-1,12-diaminododecane,
N,N'-diformyl-N,N'-di[1-methyl-2-benzyl-dithio-4-hydroxybut-1-enyl]-1.12 diaminododecane,
N,N'-diformyl-N,N'-di[1-methyl-2-(2-hydroxyethyl)-dithio-4-hydroxybut-1-enyl]-1,12-diaminododecane,
N,N'-diformyl-N,N'-di[1-methyl-2-propyldithio-4-methoxybut-1-enyl]-1,12-diaminododecane,
and N,N'-diformyl-N,N'-di[1-methyl-2-propyldithioethenyl]-1,12-diaminododecane.

13. Precursors according to claim 8, **characterized in that** they correspond to formula IV and are chosen from 2,17-(N,N'-diformyl-N,N'-dimethyl)diamino-3,16-S-thio-p-methoxybenzoyl-6,13-dioxaoctadeca-2,16-diene, 2,17-(N,N'-diformyl-N,N'-dibenzyl)diamino-3,16-S-thio-p-methoxybenzoyl-6,13-dioxaoctadeca-2,16-diene, ethyl 3,18 (N,N'-diformyl-N,N'-dimethyldiamino-4,17-S-thiobenzoyl-eicosa-3,17-dienedioate (TE12), ethyl 3,18-(N,N'-diformyl-N,N'-dibenzyl)diamino-4,17-S-thiobenzoyl-eicosa-3,17-dienedioate.

14. Precursors according to claim 8, **characterized in that** they correspond to formula (V) and are chosen from 2,15-(N,N'-diformyl-N,N'-dimethyl)diamino-1,16-S-thiobenzoyl-hexadeca-1,15-diene, 2,15-(N,N'-diformyl-N,N'-dibenzyl)diamino-1,16-S-thiobenzoyl-hexadeca-1,15-diene.

15. The cyclized derivatives corresponding to the precursors of thiazolium according to any one of claims 8 to 14 corresponding to general formula (VI) 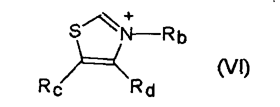 in which
. R_{b} represents R₁ or T, T representing the group of formula 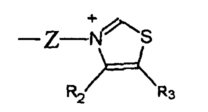 on the condition that Z does not represent a C6 to C8 alkyl radical, when R_{c}, R_{d}, R₂ and R₃ represent a methyl radical, or when R_{c} and R_{d} on the one hand, and R₂ and R₃ on the other hand, together form aromatic rings with 6 carbon atoms,
. R_{d} represents R₂ or P, P representing the group of formula 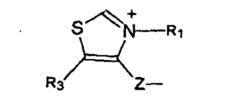
. R_{c} represents R₃ or U, U representing the group of formula 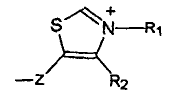
R₁, R₂, R₃ and Z being as defined in claim 1,
it being understood that R_{b} = T, if R_{c} = R₃ and R_{d} = R₂; R_{d} = P, if R_{c} = R₃ and R_{b} = R₁; and R_{c} = U, if R_{b} = R₁, and R_{d} = R₂.

16. Process for obtaining precursors of thiazolium of general formula (III) or (IV) according to claim 8, **characterized in that** it comprises the reaction in basic medium of a thiazole derivative of formula (VI).

17. Process according to claim 16, **characterized in that** in order to obtain the compounds in which Rₐ = RCO-, a derivative of thiazolium of formula (VI) is reacted with an RCOR' derivative, where R is as defined in claim 1 and R' is a halogen atom, and in order to obtain the compounds in which Rₐ = RS-, said thiazolium derivatives of formula (VI) are reacted with a thiosulphate derivative RS₂O₃Na.

18. Process according to claim 16 or 17,
**characterized in that**
- in order to obtain the compounds of formula (III) a thiazole derivative suitably substituted with an alkyl dihalide is reacted, under reflux in an organic solvent, the opening of the thiazolium ring then takes place in basic medium, and by the action either of R-COCl, or of R-S₂O₃Nₐ,
- in order to obtain the compounds of formula IV, which comprise an oxygen in the Z chain, a thiazole derivative of general formula (VII) 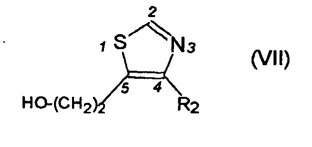 is reacted with an alkane dihalide, in basic medium, then the addition of R₁X, the reaction medium being advantageously taken to reflux in an organic solvent, in particular alcoholic such as ethanol, for a duration sufficient to obtain the quaternization of the nitrogen atom of the thiazole by fixing R₁, the opening of the thiazolium ring then being obtained in basic medium, then by the action either of R-COCl, or of R-S₂O₃Na,
- in order to obtain the compounds of formula (IV) not comprising oxygen in the Z chain, a compound of structure 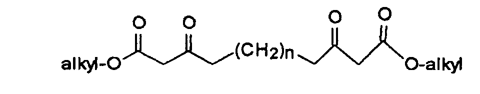 is firstly synthesized by reacting an alkyl acetoacetate with NaH, followed by alkylation, then the addition of a dihalogenoalkane, the compound obtained then being dibrominated, then thioformamide is added and, after reflux for several days, R₁X, which leads, after renewed reflux for several days, to a thiazolium, the opening of which is then carried out in basic medium, then the action of R-COCl or of R-S₂O₃Nₐ,
- in order to obtain the compounds of formula (V) not comprising oxygen in the Z chain, a Z(CO-CH₂ X)₂ compound is reacted with CH(=S)NH₂, then R₁X is added, the opening of the thiazolium ring then being carried out in basic medium, then by adding R-COCl or R-S₂O₃Nₐ.

19. Process for obtaining haloalkylamines according to claim 1, **characterized in that** it comprises the alkylation of an amino alcohol of formula 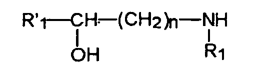 by an alkyl α,ω-dihalide X-Z-X, which leads to a bis-aminoalcohol treated with a compound capable of releasing the W group.

20. Pharmaceutical compositions, **characterized in that** they contain an effective quantity of at least one precursor as defined in any one of claims 1 to 14, or at least one cyclized derivative corresponding to thioazolium precursors of general formula (VI): 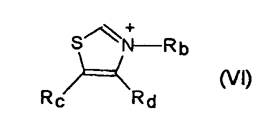 in which
. R_{b} represents R₁ or T, T representing the group of formula 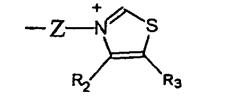
. R_{d} represents R₂ or P, P representing the group of formula 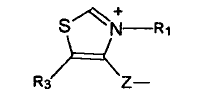
R_{c} represents R₃ or U, U representing the group of formula 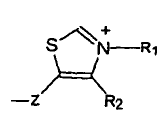 R₁, R₂, R₃ and Z being as defined in claim 1,
it being understood that R_{b} = T if R_{c} = R₃ and R_{d} = R₂ ; R_{d} = P if R_{c} = R₃ and R_{b} = R₁; and R_{c} = U if R_{b} = R₁, and R_{d} = R₂,
in combination with an inert pharmaceutical vehicle.

21. Use of quaternary bis-ammonium salts of general formula I 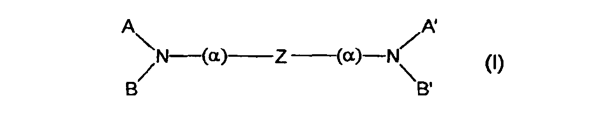 in which
- A and A' are identical to or different from one another and represent
. either, an A₁ and A'₁ group respectively of formula 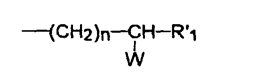 where n is an integer from 2 to 4; R'₁ represents a hydrogen atom, a C1 to C5 alkyl radical, optionally substituted by an aryl (in particular a phenyl radical), a hydroxy, an alkoxy radical, in which the alkyl radical comprises from 1 to 5 C, or aryloxy (in particular phenoxy); and W represents a halogen atom chosen from chlorine, bromine or iodine, or a nucleofuge group, such as the tosyl CH₃-C₆H₄-SO₃, mesityl CH₃-SO₃, CF₃-SO₃, NO₂-C₆H₄-SO₃ radical,
. or an A₂ group which represents a formyl -CHO, or acetyl -COCH₃ radical,
- B and B' are identical to or different from one another and represent
. either the B₁ and B'₁ groups respectively, if A and A' represent A₁ and A'₁ respectively, B₁ and B'₁ representing an R₁ group which has the same definition as R'₁ above, but which cannot be a hydrogen atom,
. or the B₂ and B'₂ groups respectively, if A and A' represent A₂, B₂ or B'₂ being the R₁ group as defined above, or a group of formula 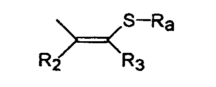 in which -Ra represents an RS- or RCO- group, where R is a linear, branched or cyclic C1 to C6 alkyl radical, optionally substituted by one or more hydroxy, alkoxy or aryloxy groups or an amino group and/or a -COOH or COOM group, where M is a C1 to C3 alkyl; a phenyl or benzyl radical, in which the phenyl radical is optionally substituted by at least one C1 to C5 alkyl or alkoxy radical, these being optionally substituted by an amino group, or by a nitrogenous or oxygenous heterocycle, a -COOH or -COOM group; or a -CH₂-heterocycle group, with 5 or 6 elements, nitrogenous and/or oxygenous; R₂ phenoxy); and W represents a halogen atom chosen from chlorine, bromine or iodine, or a nucleofuge group, such as the tosyl CH₃-C₆H₄-SO₃, mesityl CH₃-SO₃, CF₃-SO₃, NO₂-C₆H₄-SO₃ radical. represents a hydrogen atom, a C1 to C5 alkyl radical, or a -CH₂-COO-alkyl (C1 to C5) group; and R₃ represents a hydrogen atom, a C1 to C5 alkyl or alkenyl radical, optionally substituted by -OH, a phosphate group, an alkoxy radical, in which the alkyl radical is C1 to C3, or aryloxy; or an alkyl (or aryl) carbonyloxy group; or also R₂ and R₃ together form a ring with 5 or 6 carbon atoms; R and R₃ can be linked to form a ring of 5 to 7 atoms (carbon, oxygen, sulphur)
- α represents
. either a single bond, when A and A' represent A₁ and A'₁: or when A and A' represent A₂ , i.e. a -CHO or -COCH₃ group, and B₂ and B'₂ represent 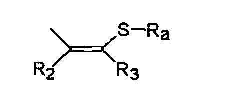
. either, when A and A' represent a -CHO group and B₂ and B'₂ represent R₁ , a group of formula 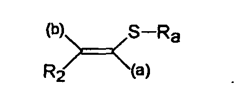 or a group of formula 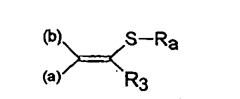 in which (a) represents a bond towards Z and (b) a bond towards the nitrogen atom,
- Z represents a C6 to C21 alkyl radical, optionally with insertion of one or more multiple bonds, and/or of one or more O and/or S heteroatoms; and/or one or more aromatic rings, and the pharmaceutically acceptable salts of these compounds
to manufacture medicaments for the treatment of infectious diseases, in particular malaria or babesiosis in humans or animals.

22. Pharmaceutical compositions according to claim 20 or medicaments manufactured according to claim 21, **characterized in that** they can be administered by oral route, by injectable route, or also by rectal route.

## Patentansprüche

1. Vorprodukte mit anti-parasitärer (Anti-Malaria) Wirkung (Prodrugs), **dadurch gekennzeichnet, daß** es sich um Produkte handelt, die bis-quaternäre Ammoniumsalze bilden können und die der allgemeinen Formel (I) entsprechen 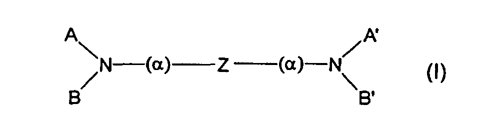
- worin A und A' gleich oder verschieden sind und das eine und das andere darstellen:
• entweder jeweils eine Gruppe A₁ und A'₁ mit der Formel 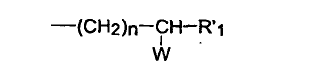 worin n eine ganze Zahl von 2 bis 4 ist; R'₁ ein Wasserstoffatom, ein Alkylrest mit C1 bis C5, der gegebenenfalls durch einen Arylrest (insbesondere einen Phenylrest) substituiert ist, ein Hydroxy, ein Alkoxy, worin der Alkylrest 1 bis 5 C-Atome aufweist, oder Aryloxy (insbesondere Phenoxy) ist und W ein Halogenatom, das ausgewählt ist aus Chlor, Brom oder Jod, oder eine nucleofuge Gruppe, wie der Tosylrest CH₃-C₆H₄-SO₃, Mesitylrest CH₃-SO₃, CF₃-SO₃, NO₂-C₆H₄-SO₃ darstellt,
• oder eine Gruppe A₂, die einen Formylrest -CHO oder Acetylrest -CO-CH₃ darstellt,
- B und B' gleich oder voneinander verschieden sind und darstellen
• entweder jeweils die Gruppen B₁ und B'₁ darstellen, wenn A und A' jeweils A₁ und A'₁ darstellen, wobei B₁ und B'₁ eine Gruppe R₁ darstellen, welche die gleiche Definition wie oben R'₁ hat, jedoch kein Wasserstoffatom sein kann,
• oder jeweils die Gruppen B₂ und B'₂ darstellen, wenn A und A' A₂ darstellen, wobei B₂ oder B'₂ die Gruppe R₁ wie oben definiert oder eine Gruppe der folgenden Formel sind: 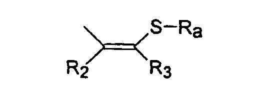 worin -Ra eine Gruppe RS- oder RCO- darstellt, worin R ein linearer, verzweigter oder cyclischer C1- bis C6-, besonders C1- bis C5-Alkylrest ist, der gegebenenfalls durch eine oder mehrere Hydroxy-, Alkoxy- oder Aryloxygruppen oder eine Amino- und/oder -COOH- oder COOM-Gruppe substituiert ist, worin M ein C1- bis C3-Alkyl ist; einen Phenyl- oder Benzylrest darstellt, worin der Phenylrest gegebenenfalls durch mindestens einen C1- bis C5-Alkyl- oder Alkoxyrest substituiert ist, wobei diese gegebenenfalls durch eine Aminogruppe oder durch einen Stickstoff- oder Sauerstoff-Heterocyclus, eine Gruppe -COOH oder -COOM oder eine Gruppe -CH₂-Heterocyclus mit Stickstoff und/oder Sauerstoff mit 5 oder 6 Elementen; R₂ ein Wasserstoffatom, einen C1- bis C5-Alkylrest oder eine Gruppe -CH₂-COO-Alkyl (C1 bis C5) darstellt und R₃ ein Wasserstoffatom, einen C1- bis C5-Alkyl- oder Alkenylrest darstellt, der gegebenenfalls durch -OH, eine Phosphatgruppe, einen Alkoxyrest, worin der Alkylrest C 1 bis C3 ist, oder Aryloxy; oder eine Alkyl- (oder Aryl-) Carbonyloxygruppe ist; oder auch R₂ und R₃ zusammen einen Ring mit 5 bis 6 Kohlenstoffatomen bilden, wobei R und R₃ verbunden sein können, um einen Ring mit 5 bis 7 Atomen (Kohlenstoff, Sauerstoff, Schwefel) zu bilden
- α darstellt
• entweder eine einfache Bindung, wenn A und A' A₁ und A'₁ darstellen, oder wenn A und A' A₂ darstellen, das heißt eine Gruppe -CHO oder -COCH₃ darstellen und B₂ und B'₂ darstellen 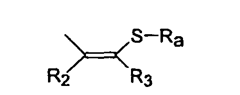
• oder wenn A und A' A₂ und B₂ und B'₂ R₁ darstellen eine Gruppe der Formel 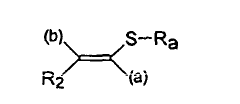 oder eine Gruppe der Formel 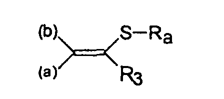 worin (a) eine Bindung zu Z und (b) eine Bindung zum Stickstoffatom darstellen,
- Z einen C6- bis C21-Alkylrest, besonders C13- bis C21-Alkykest darstellt, gegebenenfalls mit Einfügung von einer oder mehreren Doppelbindungen und/oder einer oder mehreren O- und/oder S-Heteroatomen und/oder einem oder mehreren aromatischen Ringen, und die pharmazeutisch annehmbaren Salze dieser Verbindungen, unter der Bedingung, daß R'₁ nicht H oder einen C1- oder C2-Alkylrest darstellt, wenn n = 3 oder 4, R₁ einen C1- bis C4-Alkylrest und Z einen C6- bis C10-Alkylrest darstellen.

2. Vorprodukte nach Anspruch 1, **dadurch gekennzeichnet, daß** es sich um Haloalkylamine handelt, die der allgemeinen Formel (II) entsprechen 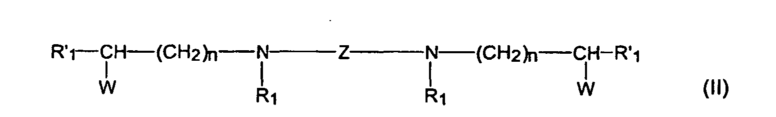 worin R₁, R'₁, W, n und Z die im Anspruch 1 definierte Bedeutung haben.

3. Vorprodukte nach Anspruch 1 oder 2, **dadurch gekennzeichnet, daß** Z einen C13- bis C21-Alkylrest darstellt.

4. Vorprodukte nach Anspruch 3, **dadurch gekennzeichnet, daß** Z eine -(CH₂)₁₆-Gruppe darstellt.

5. Vorprodukte nach einem der Ansprüche 2 bis 4, **dadurch gekennzeichnet, daß** R₁ ein Methylrest ist.

6. Vorprodukte nach einem der Ansprüche 2 bis 5, **dadurch gekennzeichnet, daß** R₁ ein Methylrest und R'₁ entweder ein Wasserstoffatom oder ein Methylrest ist und W ein Chloratom ist.

7. Vorprodukte nach einem der Ansprüche 2 bis 6, **dadurch gekennzeichnet, daß** sie ausgewählt sind aus Dichlorhydrat von N, N'-Dimethyl-N,N'-(5-chlorpentyl)-1,16-hexadecandiamin oder Dichlorhydrat von N,N'-Dimethyl-N,N'-(4-Chlorpentyl)-1,16-hexadecandiamin.

8. Vorprodukte nach Anspruch 1, **dadurch gekennzeichnet, daß** es sich um Thiazolium-Vorläufer handelt, welche der allgemeinen Formel (III) 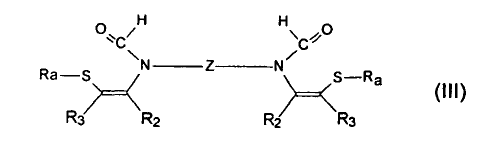 oder der allgemeinen Formel (IV) 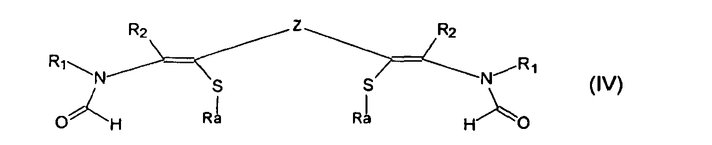 oder der allgemeinen Formel (V) 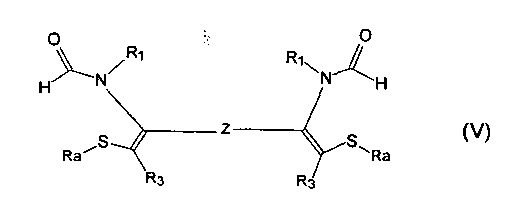 entsprechen, worin Rₐ, R₁, R₂ und Z die gleiche Bedeutung wie im Anspruch 1 haben.

9. Vorprodukte nach Anspruch 8, **dadurch gekennzeichnet, daß** sie der Formel (III) entsprechen, worin Rₐ einen RCO-Rest darstellt.

10. Vorprodukte nach Anspruch 9, **dadurch gekennzeichnet, daß** sie ausgewählt sind unter N,N'-Diformyl-N,N'-di[1-methyl-2-S-thiobenzoyl-4-methoxybut-1-enyl]-1,12-diaminododecan, N,N'-Diformyl-N,N'-di[1-methyl-2-S(p-diethylaminomethylphenylcarboxy)thio-4-methoxybut-1-enyl]-1,12-Diaminododecan, N,N'-Diformyl-N,N'-di[1-methyl-2-S-(p-morpholino-methylphenyl-carboxy)-thio-4-methoxybut-1-enyl]-1,12-diaminododecan und N,N'-Diformyl-N,N'-di[1-methyl-2-S.thiobenzoyl-4-methoxybut-1-enyl]-1, 16-Diamino-hexadecan, und N, N'-Diformyl-N,N'-di[1(2-oxo-4,5-dihydro-1,3-oxathian-4-yliden)ethyl] 1, 12-diaminododecan.

11. Vorprodukte nach Anspruch 8, **dadurch gekennzeichnet, daß** Rₐ die Bedeutung RS-hat.

12. Vorprodukte nach Anspruch 11, **dadurch gekennzeichnet, daß** sie ausgewählt sind unter N,N'-Diformyl-N,N'-di[1-methyl-2-tetrahydrofurfuryl-methyldithio-4-hydroxy-but-1-enyl]-1,12-diaminododecan, N,N'-Diformyl-N,N'-di[1-methyl-2-propyl-dithio-4-hydroxybut-1-enyl]-1,12-diaminododecan, N,N'-Diformyl-N,N'-di[1-methyl-2-benzyl-dithio-4-hydroxybut-1-enyl]-1,12-diaminododecan, N,N'-Diformyl-N,N'-di[1-methyl-2-(2-hydroxyethyl-dithio-4-hydroxybut-1-enyl]-1,12-diaminododecan, N,N'-Diformyl-N,N'-di[1-methyl-2-propyldithio-4-methoxybut-1-enyl]-1,12-diaminododecan und N,N'-Diformyl-N,N'-di[1-methyl-2-propyldithio-ethenyl]-1,12-diaminododecan.

13. Vorprodukte nach Anspruch 8, **dadurch gekennzeichnet, daß** sie der Formel (IV) entsprechen und ausgewählt sind unter 2,17-(N,N'-Diformyl-N,N'-dimethyl)diamino-3,16-S-thio-p-methoxybenzoyl-6,13-dioxaoctadeca-2,16-dien, 2,17-(N,N'-Diformyl-N,N'-dibenzyl)diamino-3,16-S-thio-p-methoxybenzoyl-6,13-dioxaoctadeca-2,16-dien, 3,18-(N,N'-Diformyl-N,N'-dimethyl)diamino-4,17-S-thiobenzoyl-eicosa-3,17-dien-ethyl-dioat (TE 12), 3,18-(N,N'-Diformyl-N,N'-dibenzyl)diamino-4,17-S-thiobenzoyl-eicosa-3,17-dien-ethyl-dioat.

14. Vorprodukte nach Anspruch 8, **dadurch gekennzeichnet, daß** sie der Formel (V) entsprechen und ausgewählt sind unter 2,15-(N,N'-Diformyl-N,N'-dimethyl)diamino-1,16-S-thiobenzoyl-hexadeca-1,15-dien, 2,15-(N,N'-Diformyl-N,N'-dibenzyl)diamino-1,16-S-thio-benzoyl-hexadeca-1,15-dien.

15. Cyclisierte Derivate, die den Thiazoliumvorläufern nach einem der Ansprüche 8 bis 14 entsprechen, mit der allgemeinen Formel (VI) 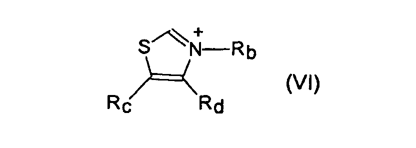
• worin R_{b} für R₁ oder T steht und T die Gruppe der Formel: 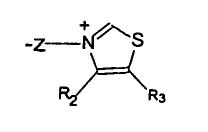 darstellt, unter der Bedingung, daß Z keinen C6- bis C8-Rest darstellt, wenn R_{c}, R_{d}, R₂ und R₃ einen Methylrest darstellen, oder wenn einerseits R_{c} und R_{d} und andererseits R₂ und R₃ miteinander aromatische Ringe mit 6 Kohlenstoffatomen bilden,
• R_{d} die Bedeutung R₂ oder P hat, worin P die Gruppe mit der folgenden Formel darstellt 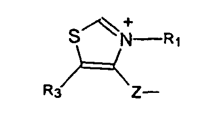
• R_{c} die Bedeutung R₃ oder U hat, wobei U die Gruppe mit der folgenden Formel darstellt 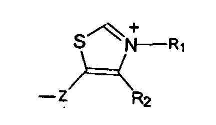
wobei R₁, R₂, R₃ und Z die gleiche Bedeutung wie im Anspruch 1 haben und R_{b} = T, wenn R_{c} = R₃ und R_{d} = R₂ sind; R_{d} = P, wenn R_{c} = R₃ und R_{b} = R₁ sind; und R_{c} = U, wenn R_{b} = R₁ und R_{d} = R₂ sind.

16. Verfahren zur Herstellung von Thiazolium-Vorprodukten der allgemeinen Formel (III) oder (IV) nach Anspruch 8, **dadurch gekennzeichnet, daß** es die Reaktion einen Thiazolderivats der Formel (VI) im basischen Milieu umfaßt.

17. Verfahren nach Anspruch 16, **dadurch gekennzeichnet, daß** man zur Gewinnung der Verbindungen, worin Rₐ = RCO- ein Thiazoliumderivat der Formel (VI) mit einem RCOR'-Derivat umgesetzt wird, worin R die im Anspruch 1 angegebene Bedeutung hat und R' ein Halogenatom ist, und daß man zur Gewinnung der Verbindungen, worin Rₐ = RS- die genannten Thiazoliumderivate der Formel (VI) mit einem Thiosulfatderivat RS₂-O₃Na reagieren läßt.

18. Verfahren nach Anspruch 16 oder 17, **dadurch gekennzeichnet, daß** man
- zur Gewinnung der Verbindungen der Formel (III) ein geeignet substituiertes Thiazolderivat mit einem Alkyldihalogenid unter Rückfluß in einem organischen Lösungsmittel reagieren läßt, wobei die Öffnung des Thiazoliumringes anschließend in basischem Milieu und durch Einwirkung von entweder R-COCl oder R-S₂O₃Nₐ erfolgt,
- zur Gewinnung der Verbindungen der Formel (IV), welche einen Sauerstoff in der Kette Z enthalten, ein Thiazolderivat der allgemeinen Formel (VII) 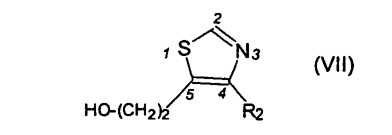 in basischem Milieu mit einem Alkandihalogenid reagieren läßt, dann R₁X zusetzt, wobei das Reaktionsmilieu vorteilhafterweise in einem organischen Lösungsmittel, insbesondere alkoholischem Lösungsmittel, wie Ethanol, während einer genügenden Zeitdauer unter Rückfluß gehalten wird, um die Quatemisierung des Stickstoffatoms des Thiazols durch Bindung von R₁ zu erhalten, wobei anschließend die Öffnung des Thiazoliumringes in basischem Milieu gefolgt von der Einwirkung von entweder R-COCl oder R-S₂O₃Na erhalten wird,
- zur Gewinnung der Verbindungen der Formel (IV), die in der Kette Z keinen Sauerstoff enthalten, man zunächst eine Verbindung der Struktur 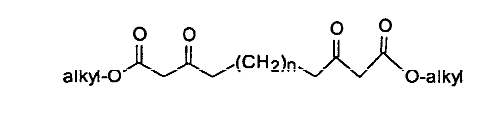 durch Umsetzung eines Alkylacetoacetats mit NaH und anschließende Alkylierung, dann Zusatz eines Dihalogenoalkans erhält, wobei die erhaltene Verbindung anschließend dibromiert wird, dann Thioformamid zugesetzt wird und nach Rückfluß während mehrerer Tage R₁X zugesetzt wird, was nach einem erneuten Rückfluß während mehrerer Tage zu einem Thiazolium führt, dessen Öffnung anschließend in basischem Milieu und dann durch R-COCl oder R-S₂O₃Nₐ durchgeführt wird,
- zur Gewinnung der Verbindungen der Formel (V), die in der Kette Z keinen Sauerstoff enthalten, man eine Verbindung Z(CO-CH₂ X)₂ mit CH(=S)NH₂ umsetzt, anschließend R₁X zusetzt, wobei die Öffnung des Thiazoliumringes anschließend in basischem Milieu erfolgt, und dann R-COCl oder R-S₂O₃Nₐ zugesetzt wird.

19. Verfahren zur Herstellung von Haloalkylaminen nach Anspruch 1, **dadurch gekennzeichnet, daß** es die Alkylierung eines Aminoalkohols der Formel 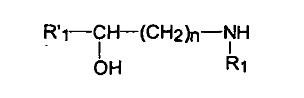 durch ein X-Z-X-Alkyl-α,ω-dihalogenid umfaßt, was zu einem bis-Aminoalkohol führt, der mit einer Verbindung behandelt wird, welche die Gruppe W freisetzen kann.

20. Pharmazeutische Zusammensetzung, **dadurch gekennzeichnet, daß** sie eine wirksame Menge mindestens eines Vorproduktes wie in einem der Ansprüche 1 bis 14 definiert oder mindestens ein cyclisiertes Derivat umfasst, das den Thiazoliumvorläufern der allgemeinen Formel (VI) entspricht 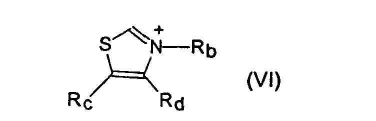
• worin R_{b} für R₁ oder T steht und T die Gruppe mit der folgenden Formel darstellt 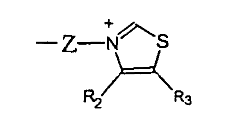
• R_{d} für R₂ oder P steht und P die Gruppe mit der folgenden Formel darstellt 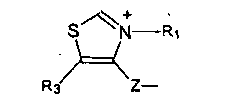
• R_{c} für R₃ oder U steht und U die Gruppe mit der folgenden Formel darstellt 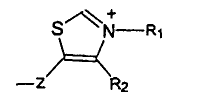 und R₁, R₂, R₃ und Z die Bedeutungen wie im Anspruch 1 haben, wobei gilt, daß R_{b} = T, wenn R_{c} = R₃ und R_{d} = R₂; R_{d} = P, wenn R_{c} = R₃ und R_{b} = R₁; und R_{c} = U, wenn R_{b} = R₁ und R_{d} = R₂, zusammen mit einem pharmazeutisch inerten Vehikel.

21. Verwendung zur Herstellung von Medikamenten zur Behandlung von Infektionskrankheiten, besonders Malaria oder Babesiosen beim Menschen oder Tier von quaternären bis-Ammoniumsalzen, die der allgemeinen Formel (I) entsprechen 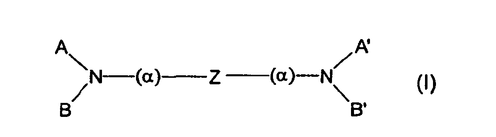
- worin A und A' gleich oder verschieden sind und das eine und das andere darstellen:
• entweder jeweils eine Gruppe A₁ und A'₁ mit der Formel 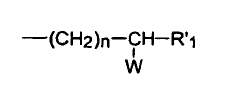 worin n eine ganze Zahl von 2 bis 4 ist; R'₁ ein Wasserstoffatom, ein Alkylrest mit C1 bis C5, der gegebenenfalls durch einen Arylrest (insbesondere einen Phenylrest) substituiert ist, ein Hydroxy, ein Alkoxy, worin der Alkylrest 1 bis 5 C-Atome aufweist, oder Aryloxy (insbesondere Phenoxy) ist und W ein Halogenatom, das ausgewählt ist aus Chlor, Brom oder Jod, oder eine nucleofuge Gruppe, wie der Tosylrest CH₃-C₆H₄-SO₃, Mesitylrest CH₃-SO₃, CF₃-SO₃, NO₂-C₆H₄-SO₃ darstellt,
• oder eine Gruppe A₂, die einen Formylrest -CHO oder Acetylrest -CO-CH₃ darstellt,
- B und B' gleich oder voneinander verschieden sind und darstellen
• entweder jeweils die Gruppen B₁ und B'₁ darstellen, wenn A und A' jeweils A₁ und A'₁ darstellen, wobei B₁ und B'₁ eine Gruppe R₁ darstellen, welche die gleiche Definition wie oben R'₁ hat, jedoch kein Wasserstoffatom sein kann,
• oder jeweils die Gruppen B₂ und B'₂ darstellen, wenn A und A' A₂ darstellen, wobei B₂ oder B'₂ die Gruppe R₁ wie oben definiert oder eine Gruppe der folgenden Formel sind: 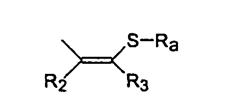 worin -Ra eine Gruppe RS- oder RCO- darstellt, worin R ein linearer, verzweigter oder cyclischer C1- bis C6-, besonders C1- bis C5-Alkylrest ist, der gegebenenfalls durch eine oder mehrere Hydroxy-, Alkoxy- oder Aryloxygruppen oder eine Amino- und/oder -COOH- oder COOM-Gruppe substituiert ist, worin M ein C1- bis C3-Alkyl ist; einen Phenyl- oder Benzylrest darstellt, worin der Phenylrest gegebenenfalls durch mindestens einen C1- bis C5-Alkyl- oder Alkoxyrest substituiert ist, wobei diese gegebenenfalls durch eine Aminogruppe oder durch einen Stickstoff- oder Sauerstoff-Heterocyclus, eine Gruppe -COOH oder -COOM oder eine Gruppe -CH₂-Heterocyclus mit Stickstoff und/oder Sauerstoff mit 5 oder 6 Elementen; R₂ ein Wasserstoffätom, einen C1- bis C5-Alkylrest oder eine Gruppe -CH₂-COO-Alkyl (C1 bis C5) darstellt und R₃ ein Wasserstoffatom, einen C1- bis C5-Alkyl- oder Alkenylrest darstellt, der gegebenenfalls durch -OH, eine Phosphatgruppe, einen Alkoxyrest, worin der Alkylrest C1 bis C3 ist, oder Aryloxy; oder eine Alkyl- (oder Aryl-) Carbonyloxygruppe ist; oder auch R₂ und R₃ zusammen einen Ring mit 5 bis 6 Kohlenstoffatomen bilden, wobei R und R₃ verbunden sein können, um einen Ring mit 5 bis 7 Atomen (Kohlenstoff, Sauerstoff, Schwefel) zu bilden
- α darstellt
• entweder eine einfache Bindung, wenn A und A' A₁ und A'₁ darstellen, oder wenn A und A'A₂ darstellen, das heißt eine Gruppe -CHO oder -COCH₃ darstellen und B₂ und B'₂ darstellen 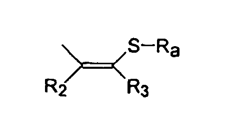
• oder wenn A und A'A₂ und B₂ und B'₂ R₁ darstellen eine Gruppe der Formel 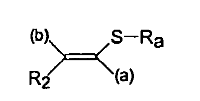 oder eine Gruppe der Formel 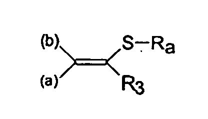 worin (a) eine Bindung zu Z und (b) eine Bindung zum Stickstoffatom darstellen,
- Z einen C6- bis C21-Alkylrest, besonders C13- bis C21-Alkylrest darstellt, gegebenenfalls mit Einfügung von einer oder mehreren Doppelbindungen und/oder einer oder mehreren O- und/oder S-Heteroatomen und/oder einem oder mehreren aromatischen Ringen, und die pharmazeutisch annehmbaren Salze dieser Verbindungen, unter der Bedingung, daß R'₁, nicht H oder einen C1- oder C2-Alkylrest darstellt, wenn n = 3 oder 4, R₁ einen C1- bis C4-Alkylrest und Z einen C6- bis C10-Alkylrest darstellen.

22. Pharmazeutische Zusammensetzung nach Anspruch 20 oder nach Anspruch 21 hergestellte Medikamente, **dadurch gekennzeichnet, daß** sie oral, durch Injektion oder auch rektal verabreichbar sind.
